(19) Europäisches Patentamt
European Patent Office
Office européen des brevets

(11) EP 0 514 490 B1

(12) EUROPEAN PATENT SPECIFICATION

(45) Date of publication and mention
of the grant of the patent:
**26.06.1996 Bulletin 1996/26**

(51) Int Cl.[6]: **C12Q 1/68**, C07H 21/04,
C12N 15/11

(21) Application number: **91905230.8**

(22) Date of filing: **07.02.1991**

(86) International application number:
**PCT/US91/00855**

(87) International publication number:
**WO 91/12339 (22.08.1991 Gazette 1991/19)**

(54) **ALLELIC ASSOCIATION OF THE HUMAN DOPAMINE (D2) RECEPTOR GENE IN COMPULSIVE DISORDERS SUCH AS ALCOHOLISM**

ALLELISCHE VERBINDUNG DES HUMANEN DOPAMIN-(D2)REZEPTOR-GENS IN KOMPULSIONSSTÖRUNGEN WIE ALKOHOLISMUS

ASSOCIATION ALLOMORPHE DU GENE RECEPTEUR DE DOPAMINE (D2) HUMAINE DANS DES TROUBLES COMPULSIFS TELS QUE L'ALCOOLISME

(84) Designated Contracting States:
**AT BE CH DE DK ES FR GB GR IT LI LU NL SE**

(30) Priority: **07.02.1990 US 477057**

(43) Date of publication of application:
**25.11.1992 Bulletin 1992/48**

(73) Proprietors:
• **BOARD OF REGENTS THE UNIVERSITY OF TEXAS SYSTEM**
  **Austin, Texas 78701 (US)**
• **THE REGENTS OF THE UNIVERSITY OF CALIFORNIA**
  **Berkeley, California 94720 (US)**

(72) Inventors:
• **NOBLE, E., P.**
  **Los Angeles, CA 90077 (US)**
• **BLUM, Kenneth, L.**
  **San Antonio, TX 78230 (US)**
• **SHERIDAN, Peter, J.**
  **San Antonio, TX 75230 (US)**

(74) Representative: **Grünecker, Kinkeldey, Stockmair & Schwanhäusser Anwaltssozietät**
  **Maximilianstrasse 58**
  **80538 München (DE)**

(56) References cited:
**WO-A-90/05780**

• **JOURNAL OF THE AMERICAN MEDICAL ASSOCIATION, vol. 263, no. 15, 18 April 1990; K. BLUM et al., pp. 2055-2060/**
• **AMERICAN JOURNAL OF HUMAN GENETICS, vol. 45, no. 5, November 1989, University of Chicago Press, IL (US); D:K: GRADY et al., pp. 778-785/**
• **JOURNAL OF THE AMERICAN MEDICAL ASSOCIATION, vol. 264, no. 24, 26 December 1990; A.M. BOLOS et al., pp. 3156-3160/**

## Description

The present invention relates to the first molecular genetic evidence, through the use of RFLP analysis, that an allele in the human dopamine ($D_2$) receptor gene is more significantly associated with human brain tissue from alcoholics than with brain tissue obtained from non-alcoholics. The occurrence of this disease-associated polymorphism has a high predictive value in the classification of, at least, one probable subtype of alcoholics.

The identification of a genetic marker that is closely linked to alcoholism means that the gene's inheritance can be followed, leading to simple tests for diagnosing carriers and future disease victims, and potential gene therapy.

The tendency of certain individuals to display compulsive behavior patterns is well known and includes individuals with an excessive desire for substances classed as psychoactive drugs including, but not limited to alcohol, opiates, and food. Whether alcoholism is a psychiatric illness or a biological disease has been a controversial question, but there is some agreement that there are probably similar biochemical mechanisms for alcohol and opiates in terms of behavioral and pharmacological activities. (1)

Some authors believe that dopaminergic cells are implicated in the rewarding action of alcohol (23), opiates (2) and cocaine (2). In contrast, others (3) argue that at least alcohol/opiates/cocaine and alcohol reinforcing effects are mediated primarily by nonadrenergic and not dopaminergic systems in the brain. Whether or not multiple systems exist, the question of several parallel reward mechanisms, or a very few, even one, is yet to be fully resolved. The cause and effect of compulsive behavior diseases, including alcoholism, appears to be biogenic. Regardless of the number of systems involved, the ability to identify an allelic gene segment associated with specific compulsive behavior is a significant step forward in developing predictive tests for compulsive behavior patterns.

Alcoholism is a major and devastating health problem with an unknown etiological basis. The question of whether environment or heredity is the prime determinant for the development of alcoholism continues to receive extensive attention throughout the world, and has recently involved the Supreme Court of the United States (4). However, family, twin, and adoption studies (5) are pointing to genetic factors as significant contributors to alcoholism. These studies also demonstrate that other forms of mental illness such as schizophrenia and other major psychoses are not found at higher frequencies in families of alcoholics compared with the general population. This would suggest that alcoholism is a primary disease, although many disagree.

Alcoholism currently is viewed as a heterogeneous entity arising from a combination of biopsychosocial factors (6). In regard to biological factors, an extensive literature reveals a wide range of potential physiological (7) and biochemical (8) markers in the risk for alcoholism. Moreover, family pedigree linkage analysis has implicated chromosomes 4, 6 and 11, but not specific gene markers, in the genetic risk for alcoholism (9).

Restriction Fragment Length Polymorphism (RFLP) offers a powerful molecular genetic tool for the direct analysis of the human genome to determine elements that provide predisposition to genetic diseases (10). This technique has been used to demonstrate a structural mutation in the gene that codes for an enzyme involved in alcohol metabolism (aldehyde dehydrogenase) which leads to the loss of this enzyme's ability to metabolize acetaldehyde. This altered gene is prevalent among Orientals (11) and may explain the well-known alcohol-flush syndrome as a protective factor in this population. However, no specific gene abnormality has been identified thus far which could regulate alcohol-seeking behavior, or is associated with alcoholism in humans.

Numerous studies indicate that, in animals, genetic control of neurotransmitter synthesis, metabolism, regulation, and receptor activity mediates reward in the meso-limbic circuitry of the brain (12), as well as drug (e.g., ethanol) - seeking behavior (13). In this regard, the dopamine$_2$ ($D_2$) receptor has been implicated as a prime target site in the N. accumbens and hippocampal $CA_1$ cluster cells of the brain reward system (14). Three major dopaminergic systems in the human brain have been identified. The nigrostriatal is involved in the initiation and execution of movement; the tuberoinfundibular is responsible for the regulation of peptide secretion from the pituitary; and the mesolimbic tract controls emotional stability and affect. Mediating these effects of dopamine are two receptor subtypes, $D_1$ and $D_2$, each of which is coupled to different second messenger systems. The $D_1$ receptor has been implicated in the sleep disorder, insomnia. Most recently, a $D_3$ receptor has been found (47) and is also implicated in limbic system function.

Important clinically relevant studies on the pharmacology of $D_2$ receptors indicated that antipsychotic drugs display high affinities for the receptor. Other work suggested that the $D_2$ receptor is involved in movement disorders, i.e., Parkinson's disease and tardive dyskinesia, tumors of the pituitary, and compulsive disease.

A cDNA encoding for rat dopamine ($D_2$) receptor has been isolated (15). This receptor has been implicated in the pathophysiology of certain diseases, including drug addiction. The same laboratory localized the receptor gene to chromosome 11 (19). Partial sequence analysis revealed that the genomic clone lambda-$hD_2G1$ ($\lambda hD_2G1$) contains the last coding exon of the $D_2$ receptor and 16.5 kb of 3-prime flanking sequence. When this clone was hybridized to human metaphase chromosomes and DNA from rodent-human hybrid cells, the data were consistent with a single human dopamine $D_2$ receptor gene which mapped to the q22-q23 region of chromosome 11. This previous work provides a research tool to begin a molecular analysis of the human $D_2$ receptor in alcoholism.

Access to sequence variation in the human genome now allows construction of genetic linkage maps through the technique of RFLPs (restriction fragment length polymorphisms). This technique provides probes which are isolated from chromosome specific phage libraries constructed to contain some portion of human DNA (16). With this tool in hand, the analysis of human gene segments is possible. The identification of an apparent gene abnormality in the tissue of alcoholics is an important advance in the art and of potential value in objectively identifying individuals who are genetically predisposed to alcoholism. The need for differential diagnosis and the ability to identify genetic predisposition for susceptibility to compulsive diseases such as alcoholism has been recognized at the national level (17).

In the present invention, the DNA probe $\lambda hD_2G1$, a 1.6 kb fragment of $\lambda hD_2G1$, and likely a 30 bit (base pair) fragment of $\lambda hD_2G1$ effectively visualize the human dopamine ($D_2$) receptor gene. This permits evaluation of polymorphisms on the gene in a region close to the gene which could modify the function of the gene as a valuable predictor of alcoholism.

In an important embodiment, the present invention concerns a method for diagnosing susceptibility to alcoholism. The method comprises initially obtaining DNA from an individual and determining the presence of a particular human $D_2$ receptor gene allele. Detection of said allele's presence in the sample is indicative of susceptibility to alcoholism, particularly because said allele has been found to be present in a large majority of clinically diagnosed severe alcoholics. The human $D_2$ receptor gene A1 allele is detected in said DNA.

The allele is readily detected by digesting DNA with Tag I restriction endonuclease and detecting the presence or absence of human $D_2$ receptor gene A1 allele in the hydrolyzed DNA. This method involves separating, preferably according to their size, restriction fragments from the digest. The separated fragments are then probed with labelled lambda-h$D_2G1$ or fragment thereof such as a Bam H1-generated 1.6 kb fragment or a synthetic 30 bit fragment of $\lambda h_2G1$ (30 base pair oligomer), for example, to specifically detect the presence of human $D_2$ receptor gene A1 allele. The presence of a 6.6 kb fragment in said sample, said 6.6 kb fragment being representative of the human $D_2$ receptor gene A1 allele, is indicative of susceptibility to alcoholism.

An object of the invention is to provide a safe and reliable method to diagnose alcohol risk at the prenatal and postnatal level.

In a particular embodiment, this method may be correlated to detect susceptibility to alcoholism with greater reliability as well as other genetic diseases such as Tourette Syndrome (48, 49, 50). This genetic disorder has been linked to a severe form of alcoholism possibly caused by a disinhibition of the limbic system (48, 49, 50).

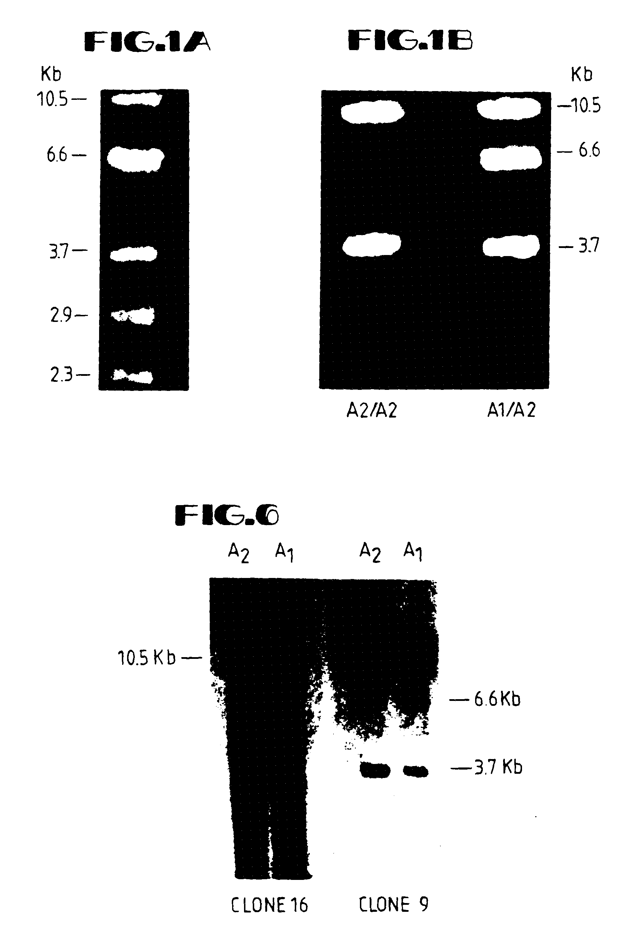

Figure 1(A) shows the hybridization pattern of Tag I-digested DNA isolated from a heterozygous individual. The hybridization probe is the full-length lambda-h$d_2G1$ which hybridizes with the 6.6 kb fragment associated with the Al allele plus the 3.7 kb and the 2.9 kb bands associated with the A2 allele. In addition, the probe also hybridizes with two constant bands, 10.5 and 2.3 kb in length.

Figure 1(B) shows the hybridization patterns of Tag I-digested DNA isolated from a nonalcoholic (homozygous for the A2 allele) and an alcoholic (heterozygous for the A1 and A2 alleles) individual. The hybridization probe is a 1.6 kb Bam H1 fragment isolated from lambda-h$D_2G1$. Note that the smaller probe does not hybridize to the 2.9 and 2.3 kb Tag I fragments of the human dopamine $D_2$ receptor gene. Lambda-h$D_2G1$ is a genomic EMBL 3 phage containing approximately 18 kb of human leukocyte DNA.

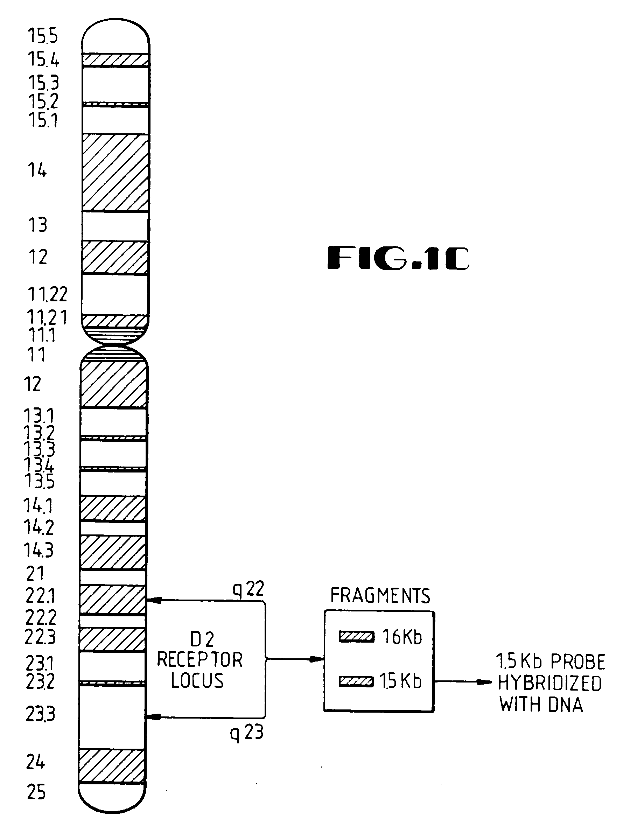

Figure 1(C) schematically shows the production of the 1.5 kb probe for the dopamine $D_2$ receptor gene obtained from chromosome 11.

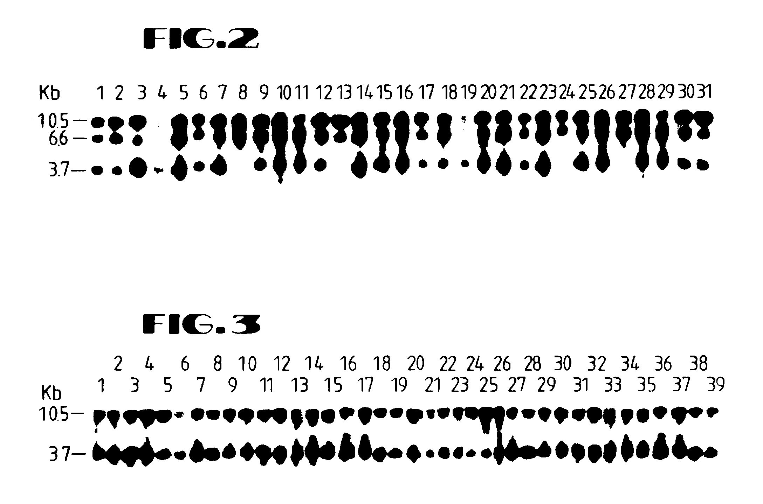

Figure 2 shows a Southern blot analysis of human DNA from brain tissue grouped according to presence of A1 allele (6.6 kb band) of the human dopamine $D_2$ receptor (D2HR) gene. Predictive value in our sample size in correctly identifying alcoholics is 77% (Chi-square=9.32, DF=1, P=0.002). Note that the A2 allele (band 3.7 kb) is missing from samples 8, 13, 24, 27, and indicating that these DNAs are homozygous for the A1 allele.

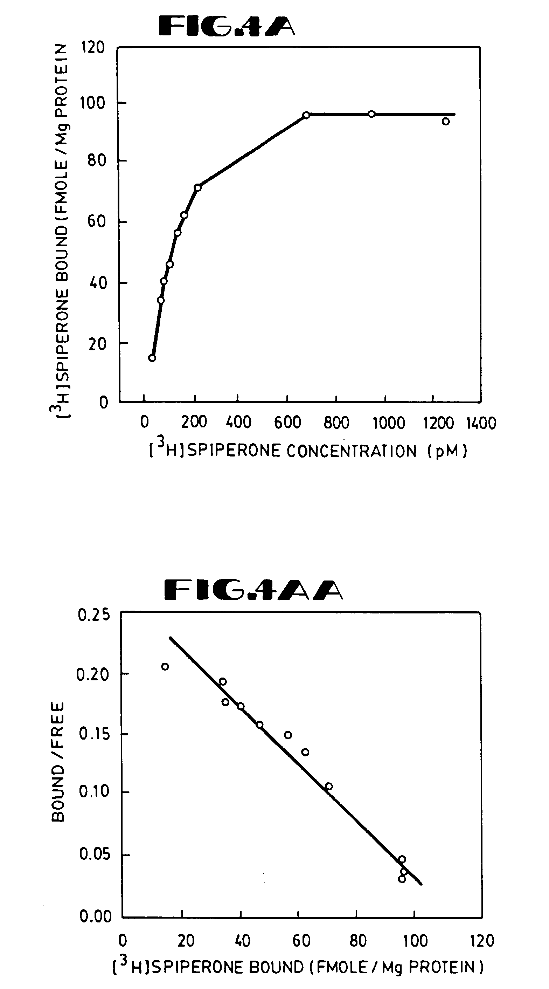

Figure 3 shows a Southern blot analysis of human DNA from brain tissue grouped according to absence of A1 allele of the human dopamine $D_2$ receptor gene. Predictive value in correctly identifying nonalcoholics is 72% (Chi-square=7.41, DF=1, P=0.002).

Figure 4 shows saturation curves of [³H]spiperone binding to D2DR in caudates of (A) a nonalcoholic subject homozygous for the $A_2$ allele (A2A2) and (B) a nonalcoholic subject heterozygous for A1 and A2 (A1A2). Data points are means of duplicate determinations. Details for the binding studies are given in Example 2. Inset: Scatchard analysis of [³H]spiperone binding.

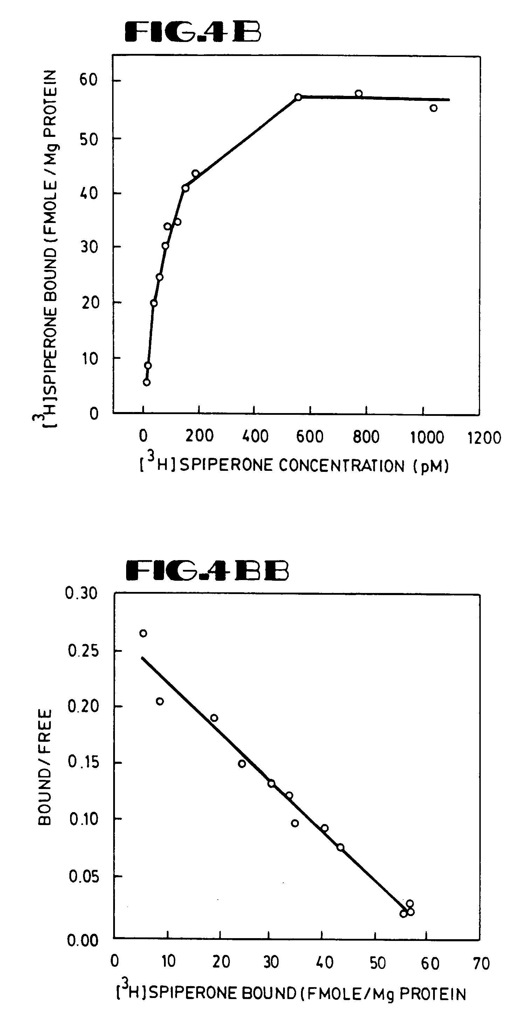

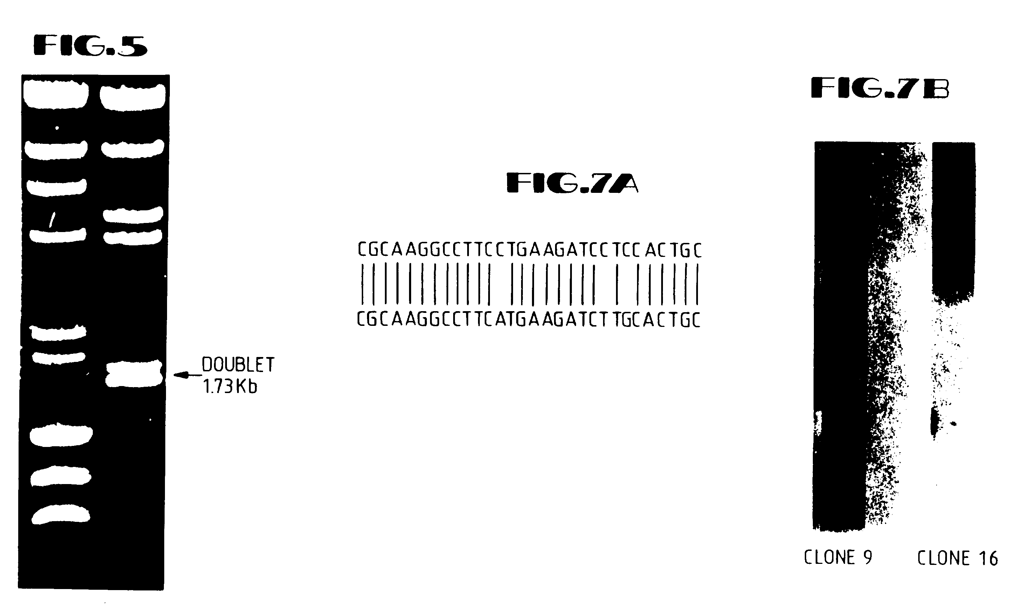

Figure 5 shows the 1.6 kb probe doublet (Doublet 1.73 kb = 1.6 kb) which results from the digestion of $\lambda hD_2G1$ with BamH1. Initially this probe was thought to consist of 1.5 kb (personal communication D.K Grandy). After sequencing the doublet was estimated as 1.73 kb. Subsequent separation of the doublet into clone 9 and clone 16 singlets, revealed, through sequencing, an actual size for the clone 9 fragment of 1.6 kb. Figure 7 illustrates the fragments from clone 9 and 16. For purposes of this application, reference to the 1.73 kb probe is equivalent to the 1.6 kb probe.

Twenty µg (ug) of the parent clone, $\lambda hD_2G1$, was digested with 48 units of BamH1 for two hours at 37°C in Buffer C (IBI), loaded onto a 0.8% agarose gel, run overnight at 23 volts, and visualized with ethidium bromide staining. The adjacent gel indicates DNA fragments of known molecular weight as standards.

Figure 6 shows the hybridization patterns of the 1.6 kb probe after doublet separation by subcloning. The 1.6 kb fragment (doublet) was ligated by following the procedure given by Sea Plaque GTG agarose and cloned as described in Example 4. DNA from antibiotic resistant clones was digested with BamH1 and separated by gel electrophoresis as in Figure 5 to identify the presence of the 1.6 kb fragment. Other digestions of clone DNA with different endonucleases (Hinf-1, Msp-1, Tag-1, BamH1, and Hind III) revealed differences in DNA patterns following separation on gel electrophoresis. In this manner, clones 9 and 16 were selected and grown. The 1.6 kb fragment from BamH1-digested DNA from clone 9 and clone 16 was radiolabeled and hybridized with Tag I-digested human genomic DNA using the same procedure as in Figure 1 above. The genomic DNA was from two individuals, one homozygous for the $A_2$ allele, indicated here as $A_2$, and one heterozygous, A1A2, indicated as $A_1$.

Figure 7 shows the nucleotide sequence and the hybridization of the 30 Base Pair Oligomer (30 bit fragment of $\lambda hD_2G1$) to the 1.6 kb probe obtained from Clone 9. The 30 base pair oligomer was custom synthesized to correspond to the last 30 base pairs of the cDNA of the 7th exon of the $D_2$ receptor. This oligomer was radiolabeled and hybridized to the 1.6 kb fragment from clone 9 but not clone 16.

## LIST OF ABBREVIATIONS

| | |
|---|---|
| IBI | International Biotechnologies, Inc. |
| A1A2 | heterozygous for the A1 and A2 allele |
| A2A2 | homozygous for the A2 allele |
| lambda-$hD_2G1$ = $\lambda hD_2G1$ | 18 kb probe used to detect A1 allele. |
| dCTP | deoxycytosine triphosphate |
| SSC | saline solution containing sodium citrate |
| SSDNA | Salmon sperm DNA |
| $D_2DR$ | Dopamine $D_2$ Receptor |
| DSM -IV-R | (criteria) diagnostic standard manual |
| $B_{max}$ | Number of receptors |
| Kd | Dissociation constant |
| TE | Tris-EDTA Buffer |
| kb = Kb | Kilobase or Kilobit |
| P | Probability |
| pM | picomolar ($10^{-12}M$) |
| fmol | femtomole ($10^{-15}$ mole) |
| RFLP | Restriction Fragment Length Polymorphism |
| ug = $\mu$g | microgram |
| ETOH | Ethanol |

## EXAMPLE I

The present invention demonstrates the first allelic association, namely of the dopamine $D_2$ receptor gene, with alcoholism. DNA, from matched alcoholic and nonalcoholic brain samples, was digested with restriction endonucleases and probed with the human $D_2$ receptor gene (lambda-$hD_2G1$). The presence of the A1 (6.6 kb band) allele of the dopamine ($D_2$) receptor (also abbreviated D2DR) gene correctly predicts 77% of alcoholics, and its absence is predictive of 72% of nonalcoholics. The polymorphic pattern of this receptor gene suggests that the abnormality in at least one form of alcoholism is located on the q22-q23 region of chromosome 11 with a co-dominant Mendelian mode of inheritance. The allelic association of the dopamine ($D_2$) receptor gene with alcoholism has a high predictive value in the classification of one probable alcoholic subtype. This subtype may represent a virulent form of alcoholism.

Seventy frozen brain samples were thawed and processed for high molecular weight genomic DNA. The tissue was homogenized in 0.25 M sucrose and a nuclear pellet prepared. Next the pellets were incubated at 37°C for three hrs in 0.05% SDS and proteinase K and the DNA was extracted with phenol, followed by extraction with chloroform: isoamyl alcohol. The DNA was then spooled out, washed with ethanol, and stored in TE at 4°C. When all the DNAs were isolated, aliquots (20 ug DNA) were digested separately with four different restriction endonucleases (i.e., Tag I, Msp I, Eco RI and Pst I) at approximately two units enzyme/ug DNA, run on agarose gels, Southern-transferred to nylon membranes, and hybridized with different DNA probes using standard methods (18). In the present experiment, the DNA samples, after digestion with the four restriction enzymes, were hybridized with a number of probes involved with either ethanol metabolism or neurotransmitter regulation of reward, including the human dopamine $D_2$ receptor gene (lambda-$hD_2G1$) to determine polymorphism. This procedure used to generate the human $D_2$ receptor gene ($\lambda hD_2G1$) is as follows: A human genomic library was screened with the rat dopamine $D_2$ receptor cDNA. The human genomic library (Clonetech) in EMBL3 was prepared from normal male leukocyte DNA and screened with a nick-translated probe containing portions of the cDNA for the coding region of the rat $D_2$ receptor. One clone, lambda-

hD$_2$G1, with an 18 kb insert was identified and characterized. This clone was found to contain the entire 3' coding exon, the polyadenylation signal, and approximately 16.4 kb of noncoding 3' sequence. Twenty μg of the parent clone (lambda-hD$_2$G1) was digested with 48 units of Bam H1 for two hrs at 37°C in Buffer C (IBI), loaded on 1% agarose gel (Sea Plaque), and run overnight at 23 volts. When digested under these conditions, several fragments were generated, including a 1.6 kb fragment and a second band of 1.5 kb, made up of two 1.5 kb fragments. The 1.5 kb band was cut, heated to 68°C, diluted by a factor of three with TE buffer, and stored at 4°C. The diluted gel was placed in boiling water for three min and then incubated for ten min at 37°C. A 25 ul aliquot was then removed and labelled to a specific activity of 1 x 10$^9$ pm/μg with [$^{32}$P]-dCTP according to the oligolabeling kit (Pharmacia). The 50 ul (microliter) incubation mixture was then chromatographed through a G-50 SEPHADEX column and the eluant used for hybridization. The Tag I digested DNAs were then transferred to Nytran membranes and hybridized with the labelled insert in 50% formamide, 5 x SSC, 1 x Denhart's, 20 mM NaH$_2$PO$_4$, 200 μg/ml of SSDNA, 0.1% SDS, 10% dextran sulfate, 0.25% dry milk, and incubated overnight at 42°C. The filters were then washed 2 x with SSC, 0.1% SDS at 55°C, and radioautographed overnight. The only endonuclease to show polymorphism with lambda-hD$_2$G1 was Tag I (vide infra).

In previous studies (19), where the lambda-hD$_2$G1 was used to probe digests of human genomic DNA, it was found that only Tag I, but not digests from 30 other endonucleases, revealed a frequent two allele RFLP. Allele A1 = 6.6 kb and allele A2 = 3.7 ± 2.9 kb with constant bands at 10.5 and 2.3 kb. Allele frequencies were measured in 43 unrelated Caucasians and calculated to have a frequency of A1= 0.24, A2 = 0.76. Co-dominant Mendelian inheritance was observed in four informative families with a total of 39 children. Additionally, the human dopamine (D$_2$) receptor gene was mapped on the q22-q23 region of chromosome 11 (19).

Figure 1 illustrates the polymorphic pattern of the human dopamine (D$_2$) receptor gene. Figure 1A depicts the polymorphic allelic pattern for the lambda-hD$_2$G1 gene clone. Figure 1B shows the allelic pattern using a BamH1 1.5 kb subclone which reduced overall background and still was informative as to the presence of alleles A1 and A2. However, the smaller probe did not hybridize to the 2.9 and 2.3 kb Tag I fragments of the human dopamine (D$_2$) gene. For illustrative purposes only, the polymorphic patterns are labelled according to their highly significant allelic association with either alcoholics (A1 allele) or nonalcoholics (the absence of A1 allele), respectively labelled A1/A2 and A2/A2. The schematic production of the 1.5 kb ("1.5 kb" illustrated here later was determined to actually be "1.6 kb" - See Example III for an explanation) subclone probe from chromosome 11 is shown in Figure 1(C).

Table 1 illustrates the polymorphic pattern of the dopamine D$_2$ receptor gene with DNA obtained from alcoholic and nonalcoholic subjects following three independent hybridizations. The A1 allele is associated with 24 of 35 (69%) known alcoholics, but it associated with only 7 of 35 (20%) nonalcoholics. In contrast, the absence of the A1 allele is associated with 28 out of 35 (80%) of nonalcoholics and with only 11 of 35 (31%) alcoholics. The proportion of the presence of the AI allele to the absence of this allele is significantly different in alcoholics as compared to nonalcoholics (Yates Chi-square corrected for continuity equals to 14.8, DF=1,P<0.001).

Table 1.

| Polymorphic pattern of the 1.5 kb fragment (lambda-hD$_2$G1) of the dopamine D$_2$ receptor gene in nonalcoholic and alcoholic brain tissue. | | |
|---|---|---|
| DNA Type | Absence of A1 Allele | Presence of A1 Allele[1] |
| Control (N = 35) | 28 (80.0)[2] | 7 (20.0) |
| Alcoholic (N = 34) | 11 (31.4) | 24 (68.6) |

[1]A1 allele = 6.6 kb

[2]Values in parenthesis represent percent of nonalcoholics or Alcoholics showing absence of the A1 allele. The proportion of the presence of the A1 allele to the absence of this allele is significantly different in alcoholics compared to nonalcoholics (Yates Chi-square corrected for continuity equals 14.8, Df=1, P<.0001).

The race of subject populations is an important determinant in allelic patterns. Recently, Kidd et al.[3] reported that at some loci, alleles that are infrequent in Caucasians are common in other populations. As the present brains were derived from both Caucasians and Blacks, the allelic frequency of the dopamine D$_2$ receptor gene was analyzed in these two racial groups.

Table 2 illustrates the Polymorphic Pattern of the Dopamine D$_2$ Receptor Gene with DNA obtained from alcoholic and non-alcoholic Caucasians and Blacks following three independent hybridizations.

[3]K. K. Kidd et al., Genome Mapping and Sequencing (Cold Spring Harbor Meeting, NY, 1989), pp. 66.

Table 2.

| Polymorphic Pattern of the Dopamine $D_2$ Receptor Gene (lambda-hD2<u>G1</u>) in Brain Tissue of Nonalcoholics and Alcoholics. | | |
|---|---|---|
| DNA Source | Absence of A1 Allele | Presence of A1 Allele[4] |
| Nonalcoholic (N=35 | 28 (80%)[5] | 7 (20%) |
| Alcoholic (N=35) | 11 (31%) | 24 (69%) |
| Nonalcoholic Caucasians (N=24) | 20 (83%) | 4 (17%) |
| Alcoholic Caucasians (N=11) | 8 (36%) | 14 (64%) |
| Nonalcoholic Blacks (N=11) | 8 (73%) | 3 (27%) |
| Alcoholic Blacks (N=13) | 3 (23%) | 10 (77%) |

[4]A1 allele = 6.6 kb.

[5]Values in parentheses represent percent of nonalcoholics or alcoholics showing absence or presence of the A1 allele.

The A1 allele is found to be associated with 14 of 22 (64%) Caucasian alcoholics, but it associated with only 4 of 24 (17%) Caucasian nonalcoholics (16). The proportion of the presence of the A1 allele to the absence of the allele in Caucasian alcoholics compared to Caucasian nonalcoholics is highly significant (Yates Chi-square=8.75, DF=1, P=0.003). In Blacks, the A1 allele is associated with 10 of 13 (77%) alcoholics, but it associated with only 3 of 11 (27%) nonalcoholics. The proportion of the presence of the A1 allele to the absence of this allele is also significantly different in Black alcoholics compared to Black nonalcoholics (Yates Chi-square=5.92, DF=1, P=0.015). Thus, in the present sample, the results favor the view that A1 allelic association is based on whether or not an individual is an alcoholic, rather than the individual's racial background.

To determine the relationship between alcoholism and the A1 allele controlling for race, we used the Mantel-Hazenzel test was used. This test evaluates the relationship between two variables, while controlling for a third. Since Chi-square=14.20 with P<0.001, there is a highly significant association between alcoholism and the A1 allele was found, even after controlling for race.

In the present sample, this test also suggested that the odds ratio of finding the AI allele in alcoholics is 8.8 times as large as that for nonalcoholics.

Figures 2 and 3 show the samples grouped according to whether or not the A1 allele was present. This grouping allowed a classification of samples based on their unique allelic association with alcoholism. Figure 2 represents 31 brain samples which possess the A1 allele (6.6). Twenty-four out of 31 DNAs that had the A1 allele were from alcoholics. This suggests, that in our sample, the predictive value of this test in correctly identifying alcoholics is 77%. Figure 3 represents 39 brain samples which did not possess the A1 allele. Since 28 out of 39 samples did not have the A1 allele and were from nonalcoholics, this suggests that the predictive value of this test in correctly identifying nonalcoholics is 72%.

To evaluate the hypothesis that the presence or absence of the A1 allele was distributed between the alcoholic or nonalcoholic groups at other than equal probabilities, a single sample Chi-square analysis with assigned expected value of 0.50 was used. When this expected value was assigned, Chi-square analysis revealed no significant difference from the expected probability for the A1 allele in nonalcoholics (Chi-square=0.47, DF-1, P=0.50). In contrast, observed frequencies were significantly different from the expected probability of 0.25 for the A1 allele in alcoholics (Chi-square equals 35.4, DF=1, P<0.001).

To evaluate the hypothesis that the presence of the A1 allele was distributed between the alcoholic or nonalcoholic groups at other than equal probabilities, a single sample Chi-square analysis with assigned expected value of 0.25 was used to approximate the frequency of the A1 allele in the general population. When this expected value was assigned, Chi-square analysis revealed no significant difference from the expected probability for the A1 allele in nonalcoholics (Chi-square=0.47, DF=1, P=0.50). In contrast, observed frequencies were significantly different from the expected probability of 0.25 for the A1 allele in alcoholics (Chi-square=35.4, DF=1, P<0.001).

Of the total alleles in the present sample, the frequency of the A1 allele was 25% and that for the A2 allele was 75%. In the samples of nonalcoholics, A1 and A2 allelic frequencies were 13% and 87% respectively. The allelic frequencies in the samples of alcoholics were: A1=37% and A2=63%. The frequency of the A1 allele in samples of nonalcoholics and alcoholics were significantly different (Yates Chi-square=9.75, DF=1, P=0.002).

To determine ability to correctly classify the alcoholic or nonalcoholic in this sample, according to the presence or absence of the A1 allele distributed between the two groups at a better than chance probability, a Chi-square analysis with assigned expected value of 0.50 was used. Observed values were significantly different from expected probability for the A1 allele (Chi-square=9.32, DF=1, P=0.002) and for the absence of the A1 allele (Chi-square=9.26, Df=1, P=0.002). These findings, taken together, suggest a strong allelic association of the dopamine $D_2$ receptor gene with alcoholism.

To determine the association of other putative genes with alcoholism, a number of additional candidate probes were used. Unlike lambda-h$D_2$G1, none of these probes revealed a polymorphic pattern of association with alcoholism.

Nuclear DNA was isolated from the matched brain samples as previously described for lambda-h$D_2$G1 probe. Twenty micrograms of DNA was digested with one of the four restriction endonucleases. The resulting DNA fragments were separated according to size by electrophoresis in 1% agarose gel, transferred to nitrocellulose membranes, fixed, and hybridized with phosphorus $^{32}$P-labelled probes. Washing of filters and autoradiography were carried out as described previously in this paper. A number of probes were employed, including alcohol dehydrogenase (pADH/3), protein kinase-C (phPKC), carboxypeptidase-A (CPA), proenkephalin (pHPE9), tryptophan hydroxylase (TPH479), tyrosine hydroxylase (BTH$_4$), monoamine oxidase B (MAOB), transferrin (TF) and others (See Table 3). Evaluation of the data (Table 3) revealed that none of these DNA probes utilizing four restriction endonucleases, (which endonucleases, to date, are responsible for about two-thirds of all known polymorphisms) are associated with alcoholism. The cDNA probe for alcohol dehydrogenase, an enzyme involved in the metabolism of alcohol, displays a polymorphism using Msp I, but the polymorphism is not linked to alcoholism. The cDNA probe for transferrin, a protein involved in hemoglobin synthesis, displays polymorphism using Eco RI, but again, this polymorphism is not associated with alcoholism. Other probes used were: protein kinase-C, involved in second messenger coupling mechanisms for neurotransmitters; carboxypeptidase-A, involved in the metabolism of the opioid peptide enkephalin; pro-enkephalin, the precursor protein for the synthesis of enkephalin; the enzyme tryptophan hydroxylase, involved in the regulation of serotonin synthesis; tyrosine hydroxylase, the rate-limiting enzyme in the synthesis of dopamine; and transferrin, a protein involved in hemoglobin dynamics. This latter group of probes as well as others displayed no polymorphism with Tag I, Msp I, Eco RI, and Pst I restriction endonucleases. Thus the only probe that showed polymorphism associated with alcoholism was lambda-$D_2$G1.

Table 3.

| Evaluations of Polymorphisms of DNA Probes with Various Endonucleases | | | | | | |
|---|---|---|---|---|---|---|
| DNA Probe | Msp I | Eco RI | Tag I | Pst I | Xba1 | Alcoholism Association |
| Alcohol Dehydrogenase(pADH/3) | yes | no | no | no | - | no |
| Protein Kinase-C(phPKC) | no | no | no | no | - | no |
| Tryptophan Hydroxylase(TPH479) | no | no | no | no | - | no |
| Pro-enkephalin(pHPE9) | no | no | no | no | - | no |
| Monoamine Oxidase(MAOB) | no | no | no | no | - | no |
| Carboxypeptidase A (CPA) | no | no | no | no | - | no |
| Transferrin(TF) | no | yes | no | no | - | no |
| Tyrosine Hydroxylase(BTH$_4$) | no | no | no | no | - | no |
| Choline Acetyl Transferase (Chat) | no | no | no | no | - | no |
| Serotonin (5HT1A) receptor (GZ1) | no | no | no | no | - | no |
| Catecholamine Receptors | | | | | | |
|     22 | no | no | no | no | - | no |
|     B1 | no | no | no | no | - | no |
|     B2 | no | no | no | no | - | no |
| GABA Receptors | | | | | | |
|     21 | no | no | no | no | - | no |
|     24 | no | no | no | no | - | no |
|     B1 | no | no | no | no | - | no |
| Dopamine B-Hydroxylase | no | no | no | no | yes | no |
| Dopamine ($D_2$) Receptor lambda-h$D_2$G1 | no | no | yes | no | - | yes |

Over the past three decades, research concerned with the interaction of genetic and environmental factors in the development of alcoholism shows that the risk for this behavior is determined by genetic as well as by environmental factors (20). However, the conclusion that there is a significant genetic component to alcoholism has led to the realization that individuals who are at risk of becoming alcoholic, because of inherited factors, are biologically different from individuals who have few or no inherited factors that predispose them to alcoholism. This notion has stimulated an extensive search for alcoholism genes (alcogenes) or markers to identify individuals at increased risk for alcoholism, a concept elaborated from studies of inbred strains of mice, C57 and DBA, with a differing predilection to alcohol (21).

It is theoretically possible that the polymorphism of the dopamine ($D_2$) receptor gene in the brains of alcoholics is due to alcohol-induced alteration in DNA (22); hence, the polymorphism observed might be a consequence of prolonged alcohol consumption by the alcoholic and thus represent a state marker instead of a trait marker. This possibility is unlikely, given the fairly wide prevalence (24%) of the A1 allele in the general population. Moreover, the presence of the A1 allele and its co-dominant Mendelian inheritance (19) in alcohol-naive children indicate that alcohol per se was not responsible for this genetic variation. It is of further interest to note that naive inbred alcohol-preferring rats show a significantly lower dopamine ($D_2$) receptor binding activity than naive alcohol-avoiding rats (23), suggesting an abnormality in this gene or in its expression. These observations support the idea that the allelic association of the dopamine ($D_2$) receptor gene, or a gene close to it, in brain tissue of alcoholics is a likely candidate trait marker for, at least, one important subtype of potential alcoholism.

Given the evidence that children of alcoholics are at a greater risk of developing alcoholism than children of non-alcoholics (24), it may be predicted that the prevalence of a candidate trait marker would be significantly greater in a population of subjects who have a positive rather than a negative family history of alcoholism.

In the present sample, derived from 70 deceased individuals, a strong association between alcoholism and the A1 of a Taq I polymorphism close to the dopamine $D_2$ receptor gene has been found. That this association prevailed in a subsample of Caucasians and Blacks raises interesting questions about the prevalence of the A1 allele in other samples of alcoholics. It is, however, important to note that a large majority of alcoholics in the present study had experienced repeated treatment failures in their alcoholic rehabilitation and whose cause of death was primarily attributed to the chronic damaging effects of alcohol on their bodily systems. It is possible then that the A1 allele found in this study may be associated with a particular subgroup of virulent alcoholism. Besides these molecular genetic studies, we have also carried out, in the same brain samples as above, the actual characteristics of the dopamine $D_2$ receptor using [$^3$H]spiperone (a dopamine [$D_2$] receptor antagonist ligand). The data show that the affinity of the dopamine ($D_2$) receptor ligand is significantly different in subjects having the A1 allele compared to those having the A2 allele. Thus, the evidence, put together, shows not only that a strong association is found between the A1 allele and alcoholism, but that the A1 and A2 allele express themselves in different dopamine ($D_2$) characteristics in the brain.

Unlike genetic diseases such as Huntington's chorea and cystic fibrosis (10), where a single gene is responsible for its expression, the heterogeneous nature of alcoholism may not allow for the generation of a single marker that can identify all individuals at risk.

Given that there are various subtypes of alcoholics (51, 52), it would have been surprising if a 100% association was found between the A1 allele and alcoholism. In this regard, the 31% of alcoholics in this study which did not associate with the dopamine ($D_2$) receptor gene polymorphism suggests some interesting possibilities: 1) environmental (25) rather than genetic factors contributed to their alcoholism; 2) other genes may be critical for the predisposition and subsequent expression of alcohol-seeking behavior. This possibility is intriguing, since it suggests that gene-specific subtypes of alcoholism could now be identified through RFLP analysis and provide the basis for multiple etiologies; and 3) there may be only partial linkage disequilibrium between the RFLP and the gene responsible for the disease. This could occur because of occasional crossover between marker and gene.

Support for alcoholism subtypes can be found in various neurochemical hypothesis, including: 1) individual differences in nerve cell membrane sensitivity to ethanol (26); 2) inherited variations in the sensitivity of sodium-potassium ATPase inhibition to ethanol (27); 3) inherited variations in neurotransmitter release and uptake systems involved in a reward cascade of events (28); 4) inherited variations in the production of abnormal amounts of tetrahydroisoquinolines (29); 5) inherited variations in the neuroadaptive mechanisms for reinforcing certain behaviors (30); and 6) inherited variation in second messenger response coupling mechanisms (31).

## EXAMPLE II

The allelic association of the human $D_2$ dopamine receptor (D2DR) gene with the binding characteristics of the D2DR was determined in 66 brains of alcoholic and nonalcoholic subjects. In a blinded experiment, the DNA from these samples was treated with the restriction endonuclease TaqI and probed with a 1.5 kb fragment from a BamH1 digest of $\lambda$h$D_2$G1. As discussed in Example I, $\lambda$h$D_2$G1 contains the entire last exon of the D2DR gene, the polyadenylation signal, and approximately 16.4 kilobases of noncoding 3' sequence of the human D2DR gene. The binding characteristics (Kd, binding affinity and Bmax, maximum number of binding sites) of the D2DR were determined in the caudate nucleus from these brains using ($^3$H)spiperone, a dopamine receptor (D2DR) antagonist ligand. Log Kd was significantly lower in alcoholic compared to nonalcoholic subjects. Moreover, a linear relationship in reduced Bmax was found respectively in A2A2, A1A2, and A1A1 allelic subjects. In individuals with the A1 allele, where a high association with alcoholism was found, the maximum number of D2DRs ($B_{max}$) was significantly reduced when compared to the $B_{max}$ in individuals with the A2 allele (53).

## METHODS

### Brain samples

Tissues from 33 alcoholic and 33 nonalcoholic subjects was obtained from the National Neurological Research Bank at the VA Medical Center, Wadsworth, Los Angeles. Frontal grey cortex and caudate nucleus were removed from the brain at autopsy by a neuropathologist and immediately frozen at -70°C until used. The 66 brains analyzed consisted of the 70 that were previously studied in Example I (32); 4 caudates were not available. The ages (average ± SEM) of the alcoholics and nonalcoholics, respectively, were 50.4 ± 2.3 years and 53.2 ± 2.6 years. The racial distribution of alcoholics included 21 whites and 12 blacks, and there were 24 white and 9 black nonalcoholics. The sex distribution of alcoholics included 30 males and 3 females, and there were 29 male and 4 female nonalcoholics. The autolysis times (average ± SEM) of the alcoholics' and nonalcoholics' brain samples were, respectively, 23.0 ± 1.5 hours and 22.6± 1.7 hours. alcoholic (Alcohol Dependence and Alcohol Abuse, using DSM-III-R criteria [33]) and nonalcoholic diagnoses were made independently by two trained psychiatrists, through examination of medical and autopsy records, interviews of treatment center personnel and relatives and alcohol consumption data. There was a 100% concordance in diagnosing alcoholic and nonalcoholic subjects between these two assessments. Examination of medical records and/or analysis of body fluids at autopsy did not reveal any of the subjects to have used neuroleptics. The cause of death included: accidents, gun-shot wounds, myocardial infarction, heart failure, cancer, gastrointestinal bleeding, suicide, and pneumonia. Informed consent was obtained from next of kin to carry out the present study.

### DNA Probe

The DNA probe, as previously used in Example I (32), was a 1.73 kb band obtained as a doublet from a <u>Bam</u>H1 digest of a human genomic fragment, λhD2G1, provided by O. Civelli. This fragment contains the last coding (7th) exon of the D2DR gene and part of 16.5 kb of 3' flanking sequence (19,34). The 1.5 kb (doublet) probe was labeled by random-priming with $[^{32}P]dCTP$ (18) to a specific activity of 1 x $10^9$ cpm/μg.

### DNA Isolation and Southern Blot Analysis

The 66 frozen brain cortical samples were coded without reference to their group identity (alcoholic and nonalcoholic). They were then thawed and processed for high molecular weight genomic DNA and hybridized by established procedures (18), as previously detailed in Example I (32).

### $D_2$ Dopamine Receptor Assay

Sixty-six frozen caudate nuclei, of the same brains from which cerebral cortex DNAs were isolated, were also coded without reference to their group identity, and assayed during a one month period for D2DR characteristics. The frozen samples were routinely ground into a fine powder in liquid nitrogen using a mortar and pestle and stored at -70°C from which small amounts of homogeneous powdered tissue could be used for assays at different times. A sample of 200-300 mg of the powdered tissue was homogenized in 30 mL of ice-cold buffer (50 mmol/L Tris-HCl, pH 7.4; 120 mmol/L NaCl; 2 mmol/L $MgCl_2$) with a Brinkman Polytron. The homogenate was centrifuged at 35,000 x g for 20 minutes. The pellet was resuspended in 30 mL of buffer and again centrifuged at 35,000 x g for 20 minutes. The final pellet was resuspended in 30 mL of buffer for the binding assay.

D2DR binding was measured by a slight modification of previously established procedures (35-37). Saturation curves (Figure 4) were obtained using 12 duplicate increasing concentrations (10-1000 pmol/L) of $[^3H]$spiperone (32.4 Ci/mmol; New England Nuclear) in buffer containing 50 mmol/L Tris-HCl, pH 7.4; 120 mmol/L NaCl; 2 mmol/L $Mgcl_2$. To measure nonsaturable binding, S-(-)-sulpiride was added to a final concentration of 10 μmol/L. Binding was initiated by the addition of membrane preparation (250-350 μg protein), and the samples were incubated in the dark at 20°C for 2 hours. Final assay volume was 1 mL. The samples were then rapidly filtered through GF/B glass fiber filters with a Brandel cell harvester. The filters were washed twice with 2 mL of ice-cold assay buffer and placed in scintillation minivials with 4 mL of scintillation fluid (National Diagnostics) for counting. Protein concentrations were determined using bovine serum albumin as the standard (38). Maximum number of binding sites ($B_{max}$) and equilibrium dissociation constants ($K_d$) were estimated using the weighted nonlinear least-squares curve-fitting (39) program LIGAND. Data were fit for both one-site and two-site models with the two-site model accepted only if a stititistically significant improvement was obtained over the one-site model.

**Statistical Analysis**

The frequency distributions for $B_{max}$, $K_d$ and $B_{max}/K_d$ were examined for departures from normality. Correlations were estimated for $B_{max}$ with age, Log $K_d$ with $B_{max}$, and $B_{max}/K_d$ with age. Mean differences between groups for $B_{max}$, Log $K_d$ and $B_{max}/K_d$ were tested using two factor ANCOVA to determine the statistical significance of the main effects and the interactions of allele and the presence or absence of alcoholism. Measures for $B_{max}$ were covariate-adjusted for and and Log $K_d$ while Log $K_d$ measures were covariate-adjusted for $B_{max}$. A one-tailed alpha criteria of < 0.05 was used to evaluate all effects upon measures of $B_{max}$ and Log $K_d$. In this study, the more powerful one-sided tests were used when the sample mean fell in the expected direction (40). No directional expectations were established for $B_{max}/K_d$, therefore, alpha criteria were set at alpha = 0.05, two-tailed.

The expression of the A1 allele consists of both homozygote and heterozygote individuals. These two groups were labeled A1A1 for homozygotes and A1A2 for heterozygotes and were compared with A2A2 homozygote individuals using single factor ANOVA and polynomial tests for trends of $B_{max}$, Log $K_d$ and $B_{max}/K_d$.

**RESULTS**

**DNA Analysis**

In the previous hybridization study in Example I (32), using $\underline{Taq}$I-digested human DNA, we have shown that a $\underline{Bam}$H1 1.6 kb doublet of the D2DR gene (λhD2G1) reduced overall background and was still informative as to the presence of D2DR gene alleles. Three bands were obtained: a constant 10.5 kb band, a 6.6 kb A1 allele and a 3.7 kb A2 allele. Using the $\underline{Bam}$H1 1.6kb doublet as probe, $\underline{Taq}$I digested DNAs from the caudate nucleus of alcoholic and nonalcoholic brains in the present study were subjected to two independent hybridizations. The results are shown in Tables 4A and 4B. Table 4A presents the 29 DNAs that show the presence of the A1 allele (A1$^+$). Twenty-two (76%) of these DNAs associated with alcoholic subjects and 7 (24%) associated with nonalcoholics. Table 4b shows the 37 DNAs that exhibit the absence of the A1 allele (A1$^-$). Eleven (30%) of these DNAs associated with alcoholic subjects and 26 (70%) associated with nonalcoholics. The proportion of the presence of the A1 allele to the absence of this allele is significantly different in alcoholics compared to nonalcoholics (Yates $\underline{X}^2$ corrected for continuity = 12.06, df = 1, P < .001). This observation, as expected, is similar to our previous study of 70 brains in Example I(32), of which 66 brains were again probed in the present investigation.

**D$_2$ Dopamine Receptor**

Saturation curves, using [$^3$H]spiperone as antagonist ligand and S-(-)-sulpiride to measure nonspecific binding, of each of the 66 caudates studied complied with a single-model binding site (39). To evaluate interassay reliability, replicate determinations, on different days for the same tissue, were also made on a subset of 10 caudates from the present brain samples. Using 100% as the assigned $K_d$ or $B_{max}$ value for the first determination, the second assay revealed $K_d$ or $B_{max}$ value for the first determination, the second assay revealed $K_d = 104 \pm 7.5\%$ and $B_{max} = 103 \pm 4.3\%$. Paired $\underline{t}$-tests showed no significant difference between the two determinations. The Spearman rank-order correlation coefficients for $K_d$ and $B_{max}$ were 0.72 and 0.95 respectively. The two-tailed significance levels for $K_d$ and $B_{max}$ were P < .009 and P < .001, respectively. These data together reveal no statistically significant differences between the two independent assays, suggesting interassay reliability. Similar good interassay reliability for the binding characteristics of the D2DR in human caudates frozen at postmortem has been observed by others (36).

Figure 4 shows examples of saturation curves of [$^3$H]spiperone and Scatchard analysis in caudate tissue: (A) non-alcoholic subject with A2A2 allele and (B) nonalcoholic subject with A1A2 allele.

The $K_d$ and $B_{max}$, for each individual caudate in the present study, are shown in Table 4. Table 4A shows the binding characteristics in A1$^+$ samples (presence of the 6.6 kb band) and Table 4B depicts the values in A1$^-$ samples (absence of the 6.6 kb band). The range of values are consistent with reported results for the binding characteristics of the D2DR in human caudate tissue (42,37,41).

**Table 4. D2 dopamine receptor binding characteristics in caudates of alcoholic and nonalcoholic subjects with presence or absence of the A1 allele of the D2 dopamine receptor gene (λhD2G1)**

**A.  Presence of the A1 allele**

| Subject Number | Alcoholic (A) or Nonalcoholic (NA) | Bmax (fmol/mg protein) | Kd (pM) |
|---|---|---|---|
| 1 | A | 43.3 | 41.6 |
| 2 | A | 28.6 | 58.4 |
| 3 | A | 36.5 | 53.4 |
| 4 | A | 84.2 | 66.6 |
| 5 | A | 46.0 | 36.3 |
| 6 | A | 35.6 | 56.1 |
| 7 | A | 45.2 | 46.2 |
| 8 | A | 27.8 | 23.9 |
| 9 | NA | 56.1 | 57.4 |
| 10 | A | 137.1 | 125.7 |
| 11 | A | 59.6 | 72.1 |
| 12 | NA | 52.4 | 89.2 |
| 13 | A | 39.4 | 49.5 |
| 14 | A | 47.8 | 61.6 |
| 15 | NA | 62.0 | 74.9 |
| 16 | A | 55.9 | 62.1 |
| 17 | A | 115.5 | 124.2 |
| 18 | NA | 39.6 | 51.2 |
| 19 | A | 45.2 | 42.6 |
| 20 | NA | 55.2 | 72.9 |
| 21 | A | 78.5 | 118.7 |
| 22 | A | 77.2 | 144.0 |
| 23 | NA | 46.0 | 55.2 |
| 24 | A | 50.2 | 95.0 |
| 25 | A | 69.3 | 104.1 |
| 26 | A | 38.4 | 62.7 |
| 27 | NA | 6.6 | 69.0 |
| 28 | A | 17.8 | 56.3 |
| 29 | A | 60.3 | 87.4 |

**B.  Absence of the A1 allele**

| Subject Number | Alcoholic (A) or Nonalcoholic (NA) | Bmax (fmol/mg protein) | Kd (pM) |
|---|---|---|---|
| 1 | NA | 65.6 | 74.4 |
| 2 | A | 96.1 | 99.6 |
| 3 | A | 24.5 | 50.4 |
| 4 | NA | 120.3 | 96.7 |
| 5 | A | 85.0 | 63.7 |
| 6 | NA | 78.0 | 105.5 |
| 7 | NA | 27.3 | 47.3 |
| 8 | A | 23.4 | 45.2 |
| 9 | NA | 53.4 | 70.0 |
| 10 | NA | 122.2 | 131.7 |
| 11 | NA | 140.6 | 105.6 |
| 12 | A | 152.8 | 117.9 |
| 13 | NA | 65.8 | 69.8 |
| 14 | NA | 146.0 | 137.8 |
| 15 | NA | 123.9 | 114.7 |
| 16 | A | 99.4 | 73.3 |
| 17 | A | 67.8 | 67.8 |
| 18 | NA | 41.5 | 47.2 |
| 19 | NA | 53.4 | 70.0 |
| 20 | NA | 82.3 | 110.7 |
| 21 | NA | 58.2 | 53.3 |
| 22 | A | 78.9 | 69.4 |
| 23 | A | 37.0 | 40.6 |
| 24 | NA | 41.6 | 198.3 |
| 25 | NA | 115.0 | 143.1 |
| 26 | NA | 73.8 | 118.4 |
| 27 | A | 35.3 | 71.7 |
| 28 | NA | 86.4 | 228.0 |
| 29 | NA | 35.8 | 45.0 |
| 30 | A | 68.3 | 66.5 |
| 31 | NA | 87.8 | 95.7 |
| 32 | NA | 99.1 | 97.8 |
| 33 | NA | 92.0 | 121.8 |
| 34 | NA | 44.9 | 80.8 |
| 35 | NA | 62.5 | 140.8 |
| 36 | NA | 45.1 | 53.3 |
| 37 | NA | 23.3 | 36.9 |

Subjects 3, 9, 22 and 27 under a are homozygous for the A1 allele

In the total sample of caudates, skewness and kurtosis values for the distribution of $K_d$ were outside the limits of ± 1, so the values were re-expressed as their natural logarithms. The log-transformed values for $K_d$ distribution were within acceptable limits for the measures of skewness and kurtosis as were the non-transformed measures for $B_{max}$ and $B_{max}/K_d$.

Correlations between outcome measures were examined to determine if they were non-independent with each other, with age or with autolysis time. Linear correlations were r = -0.37 (P = 0.0023, two-tailed) for $B_{max}$ with age, and r = 0.68 (P < 0.0001, two-tailed) for Log $K_d$ with $B_{max}$. Therefore, age and Log $K_d$ were used as covariates to remove their effects from the measures of $B_{max}$. Likewise, the effects of $B_{max}$ were removed from the effects of the Log $K_d$ measures. No correlations were evident between $B_{max}$ or Log $K_d$ and autolysis time.

Table 5 compares the unadjusted and adjusted $K_d$ and $B_{max}$ in caudates of alcoholic and nonalcoholic individuals and that of A1+ and A1- allelic subjects. In Table 4a, the mean Log $K_d$ in samples of the alcoholic group, after covariate adjustment for $B_{max}$, was found to be significantly lower than that of the nonalcoholic group when tested using a two-factor ANCOVA (P = 0.023, one-tailed). No significant $B_{max}$ differences were found among these two groups. Table 5b shows the unadjusted $B_{max}$ of A1+ allelic subjects to be significantly lower (P < .008, one-tailed) than that of A1- allelic subjects. Further, Bier measures were covariate-adjusted for age as well as Log $K_d$ values and also tested using two-factor ANCOVA. The main effect for allele was still evident (P < 0.01, one tailed), with the adjusted $B_{max}$ mean of the A1+ group being smaller than that of the A1- group. No significant differences were found in $K_d$ measures when classification was based on the presence or absence of the A1 allele.

Covariate-adjusted means for $B_{max}$ and Log $K_d$ of the D2DR were also compared in four subgroups of 66 caudates derived from: A1+ non-alcoholics (n=7), A1+ alcoholics (n=22), A1- nonalcoholics (n=26) and A1- alcoholics (n=11). The differences between the alleles for the $B_{max}$ measures were parallel between alcoholics and nonalcoholics, thus no factor interaction was detected.

Comparisons of binding measures among the A2A2, A1A2 and A1A1 groups indicated differences only for measures of $B_{max}$ (P = 0.034, two-tailed). A test for polynomial trends among the three groups demonstrated a linear relationship (P = 0.01, two-tailed) with the highest means for A2A2 followed by the A1A2 samples and with the lowest for the A1A1 group. Fisher's LSD *post hoc* test found a significant (P < 0.05) difference between the A2A2 and A1A2 groups. While the A1A1 mean was the lowest, no significant differences were obtained with a sample size of four when compared with either of the two other groups.

Table 5.

| Binding characteristics of the $D_2$ dopamine receptor in caudate of alcoholic and nonalcoholic subjects (a) and as a function of the presence (+) or absence (-) of the A1 allele (b). | | |
|---|---|---|
| a. Alcoholic and nonalcoholic subjects | | |
| | Alcoholics (n=33) | | Nonalcoholics (n=33) |
| $K_d$ (pM) | | | |
| Unadjusted | 71.0 ± 5.2 | NS | 92.8 ± 7.6 |
| Log $K_d$[1] | 4.19 ± 0.06 | P < 0.023 | 4.38 ± 0.07 |
| $B_{max}$ (fmol/mg protein) | | | |
| Unadjusted | 60.8 ± 5.6 | NS | 69.8 ± 6.0 |
| Adjusted[2] | 67.9 ± 4.4 | NS | 59.5 ± 4.9 |
| b. A1+ and A1- allelic subjects | | |
| | A1+ (n=29) | | A1- (n=37) |
| $K_d$ (pM) | | | |
| Unadjusted | 70.9 ± 5.4 | NS | 90.5 ± 7.1 |
| Log $K_d$[1] | 4.31 ± 0.07 | NS | 4.27 ± 0.06 |
| $B_{max}$ (fmol/mg protein) | | | |
| Unadjusted | 53.7 ± 4.9 | P < 0.008 | 74.4 ± 5.9 |
| Adjusted[2] | 55.8 ± 5.2 | P < 0.01 | 71.7 ± 4.2 |

[1] Log-transformed and covariate-adjusted for $B_{max}$ by least-square estimations.

[2] Covariate-adjusted for Log $K_d$ and age by least-square estimations. Significance obtained by one-tailed test; NS + not significant.

TaqI digests of human DNA, probed with a clone of a human genomic fragment of the D2DR gene (λhD2G1), reveal two alleles: A1 and A2 (19). Here, like in our previous study discussed in Example 1 (32), the present inventors have shown that digests of DNA, obtained from the cerebral cortex of alcoholics and nonalcoholics, when probed with the 1.6 kb doublet of λhD2G1, revealed the A1 allele to be associated with alcoholics and its absence to be associated with non-alcoholics. Since the caudate nucleus is among brain regions with the highest expression of the D2DR, the question raised herein is whether a relationship exists among the binding characteristics of this receptor and the polymorphic pattern of the D2DR gene in caudates of alcoholic and nonalcoholic subjects.

It should be noted that the alcoholics of the present study had a very severe type of alcoholism (43). Detailed clinical records, interviews of next of kin and examination at autopsy (both macroscopic and microscopic) revealed that these alcoholics not only had a history of heavy alcohol consumption and multiple failures in their alcoholic rehabilitation but also, in a majority of them, the cause of death was attributed to the damaging effects of alcohol on their bodily systems.

That severity of alcoholism is an important determinant in A1 allelic association is shown in an analysis of a recent study by Bolos *et al.* (44) which used a different and less severe alcoholic population than the inventors' sample. In that study of blood obtained from living Caucasian subjects, patients were divided into two groups: less severe and more severe alcoholics. Using the inventors' two Caucasian groups (nonalcoholics and very severe alcoholics) and the three groups of Bolos *et al.* (44) (CEPH 'controls'[alcoholics not excluded], less severe and more severe alcoholics) a $X^2$ test for linear trend was conducted for prevalence of the A1 allele. Increasing degree of alcoholism severity was found to correspond with significant increase (P = .0002) of A1 allele prevalence (45). Moreover, Cloninger *et al* (46), utilizing a population of very severe Caucasian alcoholics similar to inventors' and controls (alcoholics rigorously excluded), found 60% of the former group and 20% of the latter group to have the A1 allele. This distribution of the A1 allele in their two groups closely corresponds to the original observation (15).

The polymorphic pattern of this gene and its differential expression of receptors suggests the involvement of the dopaminergic system in conferring susceptibility to at least one subtype of alcoholism.

## EXAMPLE III

Following the same procedure described in Example I, the parent clone, lambda-hD$_2$G1, was obtained and digested with BamH1 and run on 0.8% agarose (not Sea Plaque) and the bands visualized with ethidium bromide staining. Figure 5 indicates a doublet in the gel on the right labeled "Doublet 1.73 kb" with DNA molecular weight standards shown in the gel on the left. For equal amounts of different, unrelated fragments, one would expect the smaller fragments to stain with less intensity since fewer nucleotides would be present. Since the two bands labeled Doublet 1.73 indicated less staining intensity in the upper, larger kb band than in the lower, smaller kb band and since only one band was sometimes observed, it was suspected that the 1.73 kb fragment may consist of two, perhaps related, fragments. Hence, the 1.73 fragment was labeled Doublet 1.73 kb.

This 1.73 kb fragment was originally estimated by gel electrophoresis to 1.6 kb (personal communication from D. K. Grandy). Subsequent sequencing of the doublet determined 1.73 kb. As described in Example IV, this doublet was separated into two different fragments in clones 9 and 16. Clone 9 was sequenced and found to actually consist of 1.6 kb, which includes about 200 bases of an intron 5' to the 7th exon of the D2Dr gene, all of the 7th exon, and about 1.3 kb of the noncoding region adjoining the 3' end of the 7th exon. Thus, what is indicated in this example and in Figure 5 as 1.73 kb is actually 1.6 kb. Any reference in this application to Doublet 1.73 or a 1.73 kb fragment or probe should be considered equivalent to Doublet 1.6 or a 1.6 kb or 1.5kb fragment or probe. Therefore, for discussion purposes in this application, 1.73 kb, 1.6 kb, and 1.5 kb refer to essentially the same fragment, which has now been determined to be 1.6 kb.

## EXAMPLE IV

To check the possibility of the 1.6 kb fragment being a doublet, a ligation was performed using this fragment by following the procedure given by Sea Plaque GTG agarose (FMC BioProducts, 5 Maple Street, Rockland, ME 04841-2994 USA). Transformation of DH5α cells was performed following the Hanahan method (Hanahan, *J. Mol. Biol.* 166 (1983):557-580).

In order to differentiate between different clones, mini-preps were performed and the DNA cut with a series of enzymes (Hinf-1, Msp-1, Tag-1, BamH1, and Hind III) establishing two definite populations containing inserts of the appropriate size (1.6 kb). These two subclones, named #9 and #16, were grown and the DNA purified. The 1.6 kb fragments from each clone were labeled with $^{32}$P and used as probes to hybridize onto human genomic DNA cut with Tag1 using the same procedure described in Example 1.

Figure 6 shows that the 1.6 kb probe from clone #9 hybridized with human genomic DNA cut with Tag1 at 6.6 kb and 3.7 kb, whereas the 1.6 kb probe from clone #16 hybridized with a band at 10.5 kb. Through this subcloning of the 1.6 kb fragment, two 1.6 kb fragments were separated since the original 1.6 kb probe, suspected of comprising a doublet, hybridized with human genomic DNA cut with Tag1 gave three bands located at 10.5 kb, 6.6 kb, and 3.7 kb. Thus, the 1.6 kb fragment from clone 9 is informative as a probe for the presence of the A1 allele in human genomic DNA.

## EXAMPLE V

After the close association of alcoholism with the A1 allele was discovered, a synthetic probe was developed to be used in the screening of genomic DNA for the presence or absence of the A1 allele. The inventors had GENETIC DESIGNS, INC., 7505 S. Main Street, Houston, Texas, custom synthesize a oligomer of 30 base pairs corresponding to the last 30 base pairs of the cDNA for the 7th exon of the $D_2$ receptor (19). The oligomer was radiolabelled at its 5' terminal by incubating at 37°C for 1 hour: 2μl of $T_4$ Polynucleotide Kinase, 3μl $^{32}\gamma$ATP (6000 Ci/mM), 1μl oligomer (100 ng/μl), 2μl of 10x Polynucleotide Kinase buffer (700mM Tris-HCL, pH 7.6; 100mM $MgCl_2$; 50mM dithiothreitol), 12μl $H_2O$. After 1 hour of incubation, this mixture was passed over a lml G-50 column (gravity flow) to separate labelled 30 base pair oligomer (30 bit fragment of $\lambda hD_2G1$) from free nucleotide.

To test this 30 base pair oligomer (30 bit fragment of $\lambda hD2G1$) as a probe which would be informative for the A1 allele, it was hybridized to the 1.6 kb DNA fragments (e.g. probes) from clones 9 and 16 described in Example IV. Figure 7 shows the nucleotide sequence of this 30 base pair oligomer and the results of the hybridization with the 1.6 kb probes from clones 9 and 16. The radiolabeled, synthetic oligomer was found to hybridize to clone 9 but not to clone 16. Since the 1.6 kb probe of clone 9 hybridizes to the 6.6 kb fragment of human genomic DNA, it is likely that this 30 base pair oligomer would hybridize to the 6.6 kb fragment of human genomic DNA and similarly function as an effective probe for detecting the presence of the A1 allele.

Another 30 bit oligomer corresponding to the first 30 base pairs of the cDNA coding for the 7th exon of the D2 receptor was tested as a potential probe for the A1 allele. In contrast to the oligomer consisting of the last 30 base pairs of the 7th exon shown in Figure 7, this oligomer hybridized to the 1.6 kb BamH1 fragments (probes) from clones 9 and 16. Therefore, it does not recognize the polymorphism associated with the A1 allele.

Although the 30 bit probe indicated in Figure 7 was hybridized to the 1.6 kb fragment from clone 9 and not the 6.6 kb fragment from a human genomic DNA, those of skill in the art will recognize that the 30 bit probe will also bind to the 6.6 kb fragment since it comprises a nucleotide sequence which is complementary to the 30 bit probe. Stated another way, since the 1.6 kb probe hybridizes with the A1 allele 6.6 kb fragment from human genomic DNA, a subfragment of the 1.6 kb probe would also be expected to hybridize to complementary sequences of the A1 allele. The 30 bit probe, being a complementary subfragment of the 1.6 kb probe, would be recognized by those skilled in the art as capable of hybridizing with a complementary sequence on the 6.6 kb fragment from human genomic DNA thereby being informative as a probe for the A1 allele.

Sequences in the 7th exon of the D2DR gene have been identified that are informative for the AI allele through their ability to hybridize with the 6.6 kb fragment of human genomic DNA. Those sequences are shown in the present invention to be found in a $\lambda hD_2G1$ probe, a 1.6 kb probe of clone 9 and a 30 bit fragment which encodes for the terminal portion of the 7th exon of the D2DR gene. Since the 30 bit fragment sequence is common to both the $\lambda hD_2G1$ probe and the 1.6 kb probe of clone 9, it is likely that analogous sequences within $\lambda hD_2G1$ would also be informative as probes for the A1 allele. Certainly one would expect other subfragments of these three probes to be capable of hybridizing to the 6.6 kb fragment which identifies the A1 allele in human genomic DNA. Considering the 30 bit fragment of $\lambda hD_2G1$ and the known art for hybridization specificity with nucleic acid oligomers, it is likely that subfragments of the 30 bit fragment would demonstrate specific hybridization to the 6.6 kb fragment of human genomic DNA and thereby be informative as probes for the A1 allele.

Thus, an embodiment of the present invention would utilize a subfragment of the 30 bit fragment comprising about 10 base pairs as a probe. In further embodiments, subfragments comprising about 15, 20, or 25 nucleotide bases of the 30 bit fragment sequence shown in Figure 7 would be utilized as probes to be informative for the A1 allele in human genomic DNA.

## EXAMPLE VI

Individuals categorized as non-alcoholic, alcoholic, children of alcoholics (COA), or drug abuser (DA) were tested for the presence of the A1 allele. Subjects were assigned to a particular category after they filled out a standard chemical identification diagnostics form for substance abuse developed by Kenneth Blum, E.P. Noble and associates as well as DSMR-3 criteria assessed by a clinician (DSMR-3 form). The 1.6 kb fragment from clone 9 was used as a hybridization probe for detecting the presence or absence of the A1 allele in the subject's DNA isolated from lymphocytes. Genomic DNA from lymphocytes was obtained by the following methodology.

**ISOLATION OF GENOMIC DNA FROM LYMPHOCYTES**

1) Collect blood in (2) capped tubes without heparin.

2) Put 10-15 mls of whole blood into a polycarbonate tube (50 ml) and fill the remainder of the tube with DNA isolation solution [0.3M sucrose, 10mM Tris (pH 7.5), 5mM $MgCl_2$, 1% Triton X-100]. Mix by inversion.

3) Centrifuge at 4,000 rpm at 4°C for 10 minutes. Aspirate the supernatant into bottle containing bleach. Wash the pellet once again with the same solution. Repeat if needed until no red blood cells are apparent.

4) quickly resuspend pellet in 4.7 mls with TE and transfer into one 30 ml corex tube.

5) Slowly add 250 µl of Proteinase K (10 mg/ml in TE). Gently mix.

6) Incubate 3 hours to overnight at 37°C in a water bath. Swirl the viscous solution periodically.

7) Gently extract the DNA 1 time with an equal volume of phenol (pH 8.0) by mixing and immediately centrifuging for 10' at 5,000 rpm. Remove aqueous layer (top) carefully. Gently extract aqueous phase with 1 vol. of chloroform: isoamyl alcohol (24:1) mix gently and centrifuge immediately. Centrifuge for 10' at 5,000 rpm. Transfer aqueous layer to polypropylene tube.

8) Add 1/10 volume (0.5 ml) of 3 M sodium acetate plus total volume (5.5 ml) of cold isopropanol. Invert slowly to mix. DNA should fall out immediately.

9. Remove DNA with looped pipet. Wash DNA with 70% ETOH, air dry briefly.

10) Resuspend in 400 to 600 µl TE (pH 7.6) in 1.5 ml flip cap tube. Store at 4°C.

11) Measure the exact concentration of the DNA and analyze an aliquot by electrophoresis through a 0.3% agarose gel. The DNA should be greater than 100 kb in size and should migrate more slowly than a marker of intact bacteriophage. Store DNA at 4°C.

Hybridizations using the 1.6 kb probe of clone 9 were carried out utilizing the hybridization conditions described in Example 1. Results of these tests are summarized in Table 6.

Table 6 under "TOTAL", which summarizes the results for those subjects with and without a family history of alcoholism, Table 6 shows that there is a good correlation between the findings of Example I, where the A1 allele was detected in brain samples from deceased subjects, and the findings of this study, where the A1 allele was detected in living subjects. The previous study of Example 1, summarized in Table 2, indicated nonalcoholics with 20% A1+ (having the A1 allele) and 80% A1- (without the A1 allele), whereas Table 5 indicates nonalcoholics with 22% A1+ and 78% A1-. Similarly, the alcoholics were found in the brain tissue study of Example I to have 69% A1+ and 31% A1-, whereas in the present study with lymphocyte DNA, alcoholics were found to have 63% A1+ and 37% A1-. These findings fit very well with other recent studies such as those of Cloninger et al. (46), where a much larger number of living alcoholics and nonalcoholics were tested for the presence of the A1 allele. In the Cloninger et al. study, the A1 allele was found to be present in 20% of the nonalcoholics versus 60% of the alcoholic (Table 6: 22 % nonalcoholics versus 63% alcoholics).

In view of interviews with the subjects in this study, it is likely that the A1 allele may be predictive of attention deficit disorder with hyperactivity (ADDH). Further, coupling of a determination for ADDH with the presence of the A1 allele may allow for a more reliable detection of alcoholism susceptibility or may be of benefit in identifying a certain subtype of alcoholism. The coupling of other indicators with the presence of the A1 allele may offer a significant advance in the detection, prediction or diagnosis of a susceptibility for other compulsive disorders.

Although the data for drug abusers in this particular study did not show a dominant presence of the A1 allele, it is believed that other restriction endonucleases or related probes would show some relationship to these particular chronic

## Table 6. Allelic Association of the D2 Receptor Gene in Alcoholism using DNA obtained from Lymphocytes

| Group | Type | N | Average Age | M | F | (+) FH | (-) FH | (+) FH A₁+ | (+) FH A₁- | (-) FH A₁+ | (-) FH A₁- | TOTAL A₁+ | TOTAL A₁- | Average P Values (Yates Chi-Squares) |
|---|---|---|---|---|---|---|---|---|---|---|---|---|---|---|
| | | | | | | | | $A_1+$ | $A_1-$ | $A_1+$ | $A_1-$ | $A_1+$ | $A_1-$ | |
| A | Non-Alcoholic* | 41 | 42.6 | 19 | 22 | 27 | 14 | 26% | 74% | 14% | 86% | 22% | 78% | - |
| B | Alcoholic** | 49 | 46.3 | 35 | 14 | 44 | 5 | 66% | 34% | 40% | 60% | 63% | 37% | .003[a] |
| C | COA*** | 17 | 11.0 | 10 | 7 | 17 | 0 | 53% | 47% | - | - | 53% | 47% | .045[a] |
| D | DA**** | 18 | 37.0 | 8 | 10 | 14 | 4 | 21% | 79% | 25% | 75% | 22% | 78% | NS[a]/.009[b] |

\*     Subjects diagnosed as non-alcoholics include alcohol abuse but not dependence or less severe
\**    Subjects diagnosed as alcoholic include dependence and most severe
\***   Subjects are children of alcoholics have at least one biologic parent clinically diagnosed as alcoholic
\**** Subjects are clinically diagnosed as drug abusers and drug dependence where alcohol, if used, is not the drug of choice

(+) FH    Family history - one or more of the subjects' family members (parents or siblings) were diagnosed alcoholics

(-) FH    Family history - NO family members diagnosed as alcoholic.

a    P values determined by comparison to the "TOTAL" value for Non-Alcoholic
b    P values determined by comparison to the "TOTAL" value for the Alcoholic

drug abusers.

It is viewed as possible that the dopamine ($D_2$) receptor gene polymorphism observed herein may also be associated with predilection to other addictive diseases, such as those relating to nicotine, narcotics or other drugs.

It is believed that research dealing with the exploration of various candidate gene probes which encode elements related to the synthesis, metabolism, storage, release, and receptor activity of neurotransmitters and neuropeptides involved in brain reward might ultimately lead to multigene trait markers which can detect susceptibility of individuals with a family history of alcoholism.

It is well known that relapse is often accompanied by stressful situations. It is of interest that in an experiment using brain tissues that had been classified as having the A1 or A2 allele in a prior study, the inventors attempted to answer the question of whether the presence of the A1 allele leads to an altered number of D2 receptors in the brain. By measuring the number of dopamine D2 receptors in the caudate nucleus, an area that normally has the highest density of these receptors, the inventors found that individuals having the A1 allele had approximately 30 percent fewer D2 receptors than those with the A2 allele. Since the dopamine D2 receptor gene controls the production of these receptors, this suggests that the A1 allele causes a reduction in the number of receptors.

This finding suggests an interesting hypothesis. It is known that dopamine acts to reduce stress. When stress occurs in an individual with a normal number of dopamine receptors, dopamine is released, all of the receptors are filled, and equilibrium is restored. In an individual who has the Al allele, however, the shortage of dopamine receptors interferes with this process and equilibrium is not restored. This person may seek alcohol or other substances or stimuli that release dopamine, in the attempt to find relief and pleasure. The desired effects do not come, however, because of the shortage of receptors, and the attempt is repeated, leading to aberrant pleasure-seeking behavior. The inventors call this concept the stress-dopamine-genotype hypothesis of craving. Taken together, the findings of a high association of the A1 allele of the $D_2$ receptor gene as well as a 30% reduction of the $D_2$ receptors in A1 allele carriers suggest the importance of diagnosing alcoholism and related behaviors including stress for risk potential.

A polymorphism of the dopamine receptor gene associated with alcoholism and its potential association with other addictive diseases is taught by the present invention. The use of the λhD$_2$G1, 1.6 kb, or 30 bit probes for detecting the A1 allele polymorphism of Tag I digests are believed to exemplify one approach to detecting the D2DR gene polymorphism associated with alcoholism. The specific approach used in the present application by way of example does not preclude alternative approaches to polymorphism detection in this gene. Basically, the present disclosure teaches a polymorphism for the dopamine receptor gene associated with alcoholism which may be detected using other approaches, in addition to that exemplified in the present disclosure by the A1 allele polymorphism in Tag I digests of human genomic DNA. For example, Bolos et al. detect a polymorphism by amplifying a 3' noncoding region of the dopamine receptor gene sequence with PCR (polymerase chain reaction) and separating the amplified fragments by electrophoresis under nondenaturing conditions. This approach is stated to reveal polymorphisms that affect the secondary structure of the single DNA strands which are amplified. Thus, it is intended that this invention encompass alternative methods of detecting polymorphisms in the dopamine receptor gene which have been shown by the instant invention to be associated with alcoholism in humans.

At this time, the present findings of an allelic association of the dopamine ($D_2$) receptor gene with alcoholism suggest that a defect in this gene, or in another gene with linkage disequilibrium with it, may cause susceptibility to, at least, one type of alcoholism. Still, this finding may hold promise for specifically focused treatment and prevention strategies. Clearly, application of the discoveries and methods described herein should have great benefit for the 28 million children of alcoholics who are potentially at risk for this disease. Finally, this research, as well as other work along similar lines, should result in the destigmatization of alcoholism, and ensure that the erroneous view of it as a moral weakness should no longer be accepted by society.

The following literature citations are incorporated in pertinent part by reference herein for the reasons cited in the above text.

## REFERENCES

1. Blum et al., U.S. Patent No. 4,761,429 (August 2, 1988).

2. Wise, R.A. and Bozarth, N.A., Pharmacol. Biochem. J. Behavior 17, 239-243 (1982) "Action of Drugs of Abuse on Brain Reward Systems: An Update with Specific Attention to Opiates".

3. Amit, Z. and Brown, Z.W. Pharmacol. Biochem. J. Behavior 17, 233-238 (1982) "Actions of Drugs of Abuse on Brain Reward Systems: A Reconsideration with Specific Attention to Alcohol".

4. Supreme Court of the United States. Traynor v. Turnage, Administrator, Veterans Administration et al., and McKelvey v. Turnage, Administrator, Veterans Administration et al. (Argued Dec. 7, 1987; decided April 20, 1988).

Syllabus no. 86-22 and no. 86-737 (Washington, DC, 1988).

5. L. Kai, <u>Alcoholism in Twins: Studies on the Etiology and Sequelae of Abuse of Alcohol</u> (Almqvist and Wiksell Publ., Stockholm, 1960); D.S. Goodwin, <u>Arch. Gen. Psychiatry</u> <u>25</u>, 545 (1971); D.S. Goodwin, <u>ibid</u>. <u>36</u>, 57 (1979); C.R. Cloninger, M. Bohman, S. Sigvardsson, <u>ibid</u>. <u>38</u>, 861 (1981).

6. American Psychiatric Association, <u>Diagnostic and Statistical Manual of Mental Disorders</u>, 3rd edition (American Psychiatric Association, Washington, DC, 1987).

7. H. Begleiter, B. Porjesz, C.L. Chow, <u>Science</u> <u>211</u>, 1064 (1981); Gabrielli <u>et al</u>., <u>Psychophysiology</u> 19, 404 (1982); Pollock <u>et al</u>., <u>Arch. Gen. Psychiatry</u> <u>40</u>, 857 (1983); H. Begleiter, B. Porjesz, B. Bihari, B. Kissin, <u>Science</u> <u>225</u>, 1493 (1984); S. O'Connor, V. Hesselbrock, A. Tasman, <u>Prog. Neuropsychopharmacol. Biol. Psychiatry</u> 10, 211 (1986); S.C Whipple, E.S. Parker, E.P. Noble, <u>J. Stud. Alcohol</u> <u>49</u>, 240 (1988).

8. S. Takahashi, N. Tani, H. Yamane, <u>Folia Psychiat. Neurol. Jpn.</u> <u>30</u>, 455 (1976); A. Wiberg, C.G. Gottfried, L. Oreland, <u>Med. Biol.</u> <u>55</u>, 181 (1977); J.L. Sullivan, J.O. Cavenar, Jr., A.A. Maltbie, P. Lister, W.W.K. Zung, <u>Biol. Psychiatry</u> <u>14</u>, 385 (1979); C.J. Fowler, K.F. Tipton, A.V.P. MacKay, M.B.H. Youdim, <u>Neuroscience</u> <u>7</u>, 1577 (1982); L. Oreland <u>et al</u>., <u>J. Neural Transm.</u> <u>56</u>, 73 (1983); G. Alexopoulos, K.W. Lieberman, R.J. Frances, <u>Am. J. Psychiatry</u> <u>140</u>, 1501 (1983); A.L. von Knorring, M. Bohman, L. von Knorring, L. Oreland, <u>Acta Psychiat. Scand.</u> <u>72</u>, 51 (1985); I. Diamond, B. Wrubel, W. Estrin, A. Gordon, <u>Proc. Natl. Acad. Sci. USA</u> <u>84</u>, 1413 (1987); B. Tabakoff <u>et al</u>., <u>N. Eng. J. Med.</u> <u>318</u>, 134 (1988); D. Mochly-Rosen <u>et al</u>., <u>Nature</u> 333, 848 (1988).

9. S. Hill, D.W. Goodwin, R. Cadoret, C.K. Osterland, S.M. Doner, <u>J. Stud. Alcohol</u> <u>36</u>, 981 (1975); Y. Shigeta <u>et al</u>., <u>Pharmacol. Biochem. Behav.</u> <u>13</u> (Suppl. 1), 89 (1980); S.Y. Hill, J. Armstrong, S.R. Steinhauer, T. Baughman, J. Zubin, <u>Alcoholism</u> (NY) 11, 345 (1987).

10. Y.W. Kan., A.M. Dosy, <u>Proc. Natl. Acad. Sci. USA</u> <u>75,</u> 5631 (1978); J.S. Gusella <u>et al</u>., <u>Nature</u> <u>306</u>, 234 (1983); D.S. Gerhard <u>et al</u>., <u>Am. J. Hum. Genet.</u> <u>36</u>, 3S (1984); M.J.M. Saraiva, P.P. Costa, D.S. Goodman, <u>Neurology</u> <u>36</u>, 1413 (1986); R.J. Bartlett <u>et al</u>., <u>Science</u> <u>235</u>, 1648 (1987); P.H. St. George-Hyslop <u>et al</u>., <u>ibid</u>, 885; M. Barrow <u>et al</u>., <u>Nature</u> <u>326</u>, 289 (1987); J.M. Rommens <u>et al</u>., <u>Science</u> <u>245</u>, 1059 (1989); J.R. Riordan <u>et al</u>., <u>ibid</u>, <u>1066</u>; B. Kerem <u>et al</u>., <u>ibid</u>, <u>1073</u>.

11. W.F. Bosron, T-K. Li, <u>Biochem. Biophys. Res. Commun.</u> <u>91</u>, 1549 (1979); D.P. Agarwal, S. Harada, H.W. Goedde, <u>Alcoholism (NY)</u> <u>5</u>, 12 (1981); W.F. Bosron, L.J. Magnes, T-K. Li, <u>Biochem. Genet.</u> <u>21</u>, 735 (1983); A. Yoshida, G. Wang, V. Dave, <u>Am. J. Hum. Genet.</u> <u>35</u>, 1107 (1983); A. Yoshida, I-Y. Huang, M. Ikawa, <u>Proc Natl. Acad. Sci. USA</u> <u>81</u>, 258 (1984); H.W. Goedde, D.P. Agarwal, in <u>Genetics of Alcoholism</u>, H.W. Goedde, D.P. Agarwal, Eds. (Alan Liss, NY, 1987), pp. 3-20.

12. A.S. Lippa, S.M. Antelman, A.E. Fisher, D.R. Canfield, <u>Pharmacol. Biochem. Behav.</u> <u>1</u>, 23 (1973); L. Ahtee, K. Eriksson, <u>Acta Physiol. Scand.</u> <u>93</u>, 563 (1975); M.D. Dibner, N.R. Zahniser, B.B. Wolfe, R.A. Rabin, P.B. Molinoff, <u>Pharmacol. Biochem. Behav.</u> <u>12</u>, 509 (1980); R.A. Wise, <u>ibid</u>, <u>13</u> (Suppl. 1), 213 (1980); M.L. Barbaccia, A. Reggiani, P.F. Spano, M. Trabucchi, <u>Psychopharmacology</u> <u>74</u>, 260 (1981).

13. M.K. Ticku, T. Burch, <u>J. Neurochem.</u> <u>34</u>, 417 (1980); S.F.A. Elston, K. Blum, L. DeLallo, A.H. Briggs, <u>Pharmacol. Biochem. Behav.</u> <u>16</u>, 13 (1982); K. Blum, S.F.A. Elston, L. DeLallo, A.H. Briggs, J.E. Wallace, <u>Proc. Natl. Acad. Sci. USA</u> <u>80</u>, 6510 (1983); K. Blum, A.H. Briggs, J.E. Wallace, C.W. Hall, M.C. Trachtenberg, <u>Experientia</u> <u>43</u>, 408 (1986); C. Gianoulakis, A. Gupta, <u>Life Sci.</u> <u>39</u>, 2315 (1986); K. Blum, H. Topel, <u>Funct. Neurol.</u> <u>1</u>, 71 (1986); J.M. Murphy, W.J. McBride, L. Lumeng, T-K. Li, <u>Pharmacol. Biochem. Behav.</u> <u>26</u>, 389 (1987); J.M. Murphy <u>et al</u>., <u>Alcohol</u> <u>5</u>, 283 (1988).

14. S. Liljequist, <u>Acta Pharmacol. Toxicol.</u> <u>43</u>, 19 (1978); S.A. Newlin, J. Mancillas-Trevino, F.E. Bloom, <u>Brain Res.</u> <u>209</u>, 113 (1981); G. Mereu, F. Fadda, G.L. Gessa, <u>Brain Res.</u> <u>292</u>, 63 (1984); L. Stein, J. Belluzzi, <u>Clin. Neuropharmacol.</u> <u>9</u> (suppl. 4), 205 (1986); S. Govoni <u>et al</u>., <u>Brain Res.</u> <u>381</u>, 138 (1986); P. Valverius, P.L. Hoffman, B. Tabakoff, <u>J. Neurochem.</u> <u>52</u>, 492 (1989); F. Fadda, E. Mosca, G. Columbo, G.L. Gessa, <u>Life Sci.</u> <u>44</u>, 281 (1989).

15. Bunzow et al. <u>Nature</u>, <u>336</u>, 783-787 (1988) "Cloning and Expression of a Rat ($D_2$) Dopamine Receptor cDNA".

16. Maslen, <u>et al</u>. <u>Genomics</u>, <u>2</u>, 66 (1988).

17. Tabakoff, et al. Public Health Reports, Vol. 103(6), 690-698 (1988) "Genetics and Biological Markers of Risk for Alcoholism".

18. T. Maniatis, E.F. Fritsch, J. Sambrook, Eds., Molecular Cloning. A Laboratory Manual (Cold Spring Harbor Laboratory, Cold Spring Harbor, NY, 1982).

19. D.K. Grandy et al., Am. J. Hum. Genet., 45, 778-785 (1989).

20. C.R. Cloninger, T-K. Li, Alcoholism: An Inherited Disease (U.S. Government Printing Office, Washington, DC, 1985), DHHS Publ. No. (ADM)85-1426; C.R. Cloninger, Psychiat. Dev. 3, 167 (1986).

21. R.D. Myers, in Aldehyde Adducts in Alcoholism, M.A. Collins, Ed. (Alan R. Liss, NY, 1985), pp. 201-220.

22. G. Obe, H. Ristow, J. Herha, in Biochemistry and Pharmacology of Ethanol: Vol. I, E. Majchrowicz, E.P. Noble, Eds. (Plenum Press, NY, 1979), pp. 659-676.

23. E.R. Korpi, J.D. Sinclair, O. Malminen, Pharmacol. Toxicol. 61, 94 (1987).

24. M.A. Schuckit, in Psychiatry Update: Vol. III, L. Grinspoon, Ed. (American Psychiatric Press, Washington, DC, 1986), pp. 320-328.

25. Blocking of dopamine ($D_2$) receptors reduces reward-seeking behaviors in animals (C.R. Gallistel, A.J. Davis, Pharmacol. Biochem. Behav. 19, 867 [1983]). Acute alcohol administration increases pleasureful behavior by releasing dopamine (A. Imperato, G. Chiara, J. Pharmacol. Exp. Ther. 239, 219 [1986]) through stimulation of dopaminergic neurons in the ventral tegmental area of the brain (G.L. Gessa, F. Muntoni, M. Collu, L. Vargiu, G. Mereu, Brain Res. 348, 201 [2985]). However, chronic ethanol ingestion reduces the responsiveness and number of dopamine ($D_2$) receptors in striatal membranes of rats (L. Lucchi, R.M. Moresco, S. Govoni, M. Trabucci, Brain Res. 449, 337 [1988]). The consequence of decreased activity of this reward system may lead to a compensatory increase in alcohol-seeking behavior. This could be one nongenetic mechanism for developing alcoholism.

26. D.B. Goldstein, J.H. Chin, R.C. Lyon, Proc. Natl. Acad. Sci. USA 79, 4231 (1982).

27. Y. Israel, H. Kalant, I. Lanfer, Biochem. Pharmacol. 14, 1803 (1965); A.L. Swann, J. Pharmacol. Exp. Ther. 232, 475 (1985); P.T. Nhamburo, B.P. Salafsky, B. Tabakoff, P.L. Hoffman, Biochem. Pharmacol. 36, 2027 (1987).

28. F.E. Bloom, Advanc. Pharmacol. Ther. 2, 205 (1979); K. Blum, A.H. Briggs, M.C. Trachtenberg, Experientia 45, 444 (1989); K. Blum, Integr. Psychiat. 6, 199 (1989).

29. G. Siggins, T. Berger, E.D. French, T. Shier, F.E. Bloom, Proc. Clin. Biol. Res. 90, 275 (1982); R.D. Myers, Experientia 45, 436 (1989); R.D. Myers, T.H. Privette, Brain Res. Bull. 22, 899 (1989); B. Faraj, V.M. Camp, D.C. Davis, J.D. Lenton, K. Kutner, Alcoholism 13, 155 (1989).

30. C.R. Cloninger, M. Bohman, S. Sigvardsson, Arch. Gen. Psychiatry 38, 861 (1981); C.R. Cloninger, Science 236, 410 (1987); D.W. Goodwin, J. Stud. on Alc. 50, 397 (1989).

31. J.H. Allison, T.J. Cicero, J. Pharmacol. Exp. Ther. 213, 24 (1980); P.L Hoffman, F. Moses, G.R. Luthin, B. Tabakoff, Mol. Pharmacol. 30, 13 (1986); G.Y. Sun, H-M. Huang, R. Chandrasekhar, D.Z. Lee, A.Y. Sun, J. Neurochem. 48, 974 (1987); T. Ritchie, H-S. Kim, R. Cole, J. de Vellis, E.P. Noble, Alcohol 5, 183 (1988); D.H. Ross, Experientia 45, 407 (1989).

32. Blum K, Noble EP, Sheridan PJ, Montgomery A, Ritchie T, Jagadeeswaran P, Nogami H, Briggs AH, Cohn JB. Allelic association of human dopamine $D_2$ receptor gene in alcoholism. JAMA. 263:2055-2060 (1990).

33. Diagnostic and Statistical Manual of Mental Disorders, Third Edition. Revised. Washington, D.C.: American Psychiatric Association; (1987).

34. Grandy DK, Marchionni MA, Makam H, Stofko RE, Alfano M, Frothingham L, Fischer J, Burke-Howie KJ, Bunzow JR, Server AC, Civelli O. Cloning of the cDNA and gene for a human $D_2$ dopamine receptor. Proc Natl.

Acad. Sci. USA. 86:9762-9766 (1989).

35. Seeman P, Grigoriadis D. Dopamine $D_2$ receptor dissociation constant for spiperone: Identical value using $^3$H-labeled agonist or $^3$H-labeled antagonist. Biochem. Pharmacol. 34:4065-4066 (1985).

36. Seeman P, Bzowej NH, Guan H-C, Bergeron C, Becker LE, Reynolds GP, Bird ED, Riederer P, Jellinger K, Watanabe S, Tourtellotte WW. Human brain dopamine receptors in children and aging adults. Synapse. 1:399-404 (1987).

37. De Keyser J, Walraevens H, De Backer J-P, Ebinger G, Vauquelin G. $D_2$ dopamine receptors in the human brain: heterogeneity based on differences in guanine nucleotide effect on agonist binding, and their presence on corticostriatal nerve terminals. Brain Res. 484:36-42 (1989.

38. Lowry OH, Rosebrough NJ, Farr AL, Randall RJ. Protein measurement with the folin phenol reagent. J. Biol Chem. 193:265-275 (1951).

39. Munson PJ, Rodbard D. LIGAND: A versatile computerized approach for characterization for ligand-binding systems. Anal. Biochem. 107:220-239 (1980).

40. Rosner B. Fundamentals of Biostatistics. 2nd ed. Boston, MA: Duxbury Press; 1980:195.

41. Severson JA, Marcusson J, Winblad B, Finch CE. Agecorrelated loss of dopaminergic binding sites in human basal ganglia. J. Neurochem. 39:1623-1631 (1982).

42. Seeman P, Ulpian C, Bergeron C, Riederer P. Jellinger K, Gabriel E, Reynolds GP, Tourtellotte WW. Bimodal distribution of dopamine receptor densities in brains of schizophrenics. Science. 225:728-730 (1984).

43. Blum K, Noble EP, Sheridan PJ, Montgomery A, Ritchie T, Jagadeeswaran P, Nogami H, Briggs AH, Cohn JB. Allelic association of human dopamine $D_2$ receptor gene in alcoholism. JAMA. 263:2055-2060 (1990).

44. Bolos AM, Dean M, Lucas-Derse S, Ramsburg M, Brown GL, Goldman D. Population and pedigree studies reveal a lack of association between the dopamine $D_2$ receptor gene and alcoholism. JAMA. 264:3156-3160 (1990).

45. Noble, EP, Blum, K. Letter to the Editor. JAMA. (in press) (1991).

46. Cloninger *et al.,* Arch. Gen. Psych., In Press (1991).

47. Sokoloff P, Giros B, Martres M-P, Bouthenet M-L, Schwartz JC. Molecular cloning and characterization of a novel dopamine receptor (D3) as a target for neuroleptics. Nature (London) 347:146-151 (1990).

48. Comings DE, Comings BG. A controlled study of Tourette syndrome. I. Attention-deficit disorder, learning disorders, and school problems. Am. J. Hum. Genet. 41:701-741 (1987).

49. Comings, DE. A controlled study of Tourette syndrome. VII. Summary: A common genetic disorder causing disinhibition of the limbic system. Am. J. Hum. Genet. 41:839-866 (1987).

50. Comings, DE, Comings BG Controlled family history study of Tourette's syndrome. II: alcoholism, drug abuse and obesity. J. Clin. Psychiatry. 51:281-287 (1990).

51. Pickens RW, Svikis DS, McGue M, Lykken DT, Heston LL, Clayton PJ. Heterogeneity in the inheritance of alcoholism. Arch. Gen. Psychiatry. 48:19-28 (1991).

52. Tarter RE, McBride H, Buonpane N, Schneider DU. Differentiation of alcoholics - childhood history of minimal brain dysfunction, family history, and drinking pattern. Arch. Gen. Psychiatry 34:761-768 (1977).

53. Noble EP, Blum K, Ritchie T, Montgomery A, Sheridan, PJ. Allelic association of the $D_2$ dopamine receptor gene with receptor binding characteristics in alcoholism, Arch. Gen. Psychiatry, In Press (1991).

**Claims**

1. A method for diagnosing the susceptibility to alcoholism, the method comprising:

   detecting human dopamin $D_2$ receptor gene allele present in a sample from an individual by hybridizing the DNA of a sample from an individual to a dopamin $D_2$ receptor gene probe;
   wherein susceptibility to alcoholism of an individual may be diagnosed, when the human dopamin $D_2$ receptor gene A1 allele, identifiable by presence of a 6.6 kb Tag I fragment, is present;
   wherein the A1 allele is present in a majority of clinical diagnosed alcoholics.

2. The method for diagnosing susceptibility to alcoholism according to claim 1, the method comprising:

   determining human $D_2$ receptor gene alleles present in a sample from an individual;

   where susceptibility to alcoholism of an individual may be diagnosed when allele A1 is found.

3. The method for diagnosing susceptibility to alcoholism according to any of claims 1 or 2, the method comprising

   digesting DNA from a subject with a restriction endonuclease;

   determining human $D_2$ receptor gene allele present in said digested DNA; and

   a susceptibility to alcoholism being diagnosed when a particular allele is present,

   which is found in a majority of clinically diagnosed alcoholics.

4. The method for diagnosing susceptibility to alcoholism according to claim 3, the method comprising:

   digesting DNA from a subject with Tag I restriction endonuclease;

   determining the presence of human $D_2$ receptor gene alleles in said digested DNA;

   wherein a susceptibility to alcoholism may be diagnosed when a particular allele, which is found in a majority of clinically diagnosed alcoholics, is present.

5. The method for diagnosing susceptibility to alcoholism according to claim 3, the method comprising:

   digesting DNA from a subject with Tag I restriction endonuclease;

   separating DNA fragments found in the digest;

   probing separated fragments with labelled $\lambda hD_2G1$ or fragments thereof having about 30 bits to determine the presence of human $D_2$ receptor gene alleles in said sample; and

   where susceptibility to alcoholism may be diagnosed when a particular allele characterized as being found in a majority of clinically diagnosed alcoholics is present.

6. The method for diagnosing susceptibility to alcoholism according to any of claims 1, 3 or 4, wherein the human $D_2$ receptor gene allele is a human $D_2$ receptor gene A1 allele.

7. The method for diagnosing susceptibility to alcoholism according to claim 5, wherein said separated fragments are probed with a Bam H1-generated 1.6 kb fragment of $\lambda hD_2G1$.

8. The method for diagnosing susceptiblity to alcoholism according to claim 5, wherein said separated DNA frgaments are probed with a 30 bit fragment of $\lambda hD_2G1$.

9. A method for diagnosing susceptibility to alcoholism according to claim 3, the method comprising:

digesting DNA from a subject with Tag I restriction endonuclease;

separating DNA fragments obtained from the hydrolysate according to fragment size;

probing separated DNA fragments with labelled $\lambda hD_2G1$ or a portion of labelled $\lambda hD_2G1$ having a size of at least 30 bits;

determining the presence of a 6.6 kb DNA fragment in said separated fragments which is labelled by the probe; and

where a susceptibility to alcoholism may be diagnosed when said labelled 6.6 kb fragment is found.

10. The method for diagnosing susceptibility to alcoholism as recited in claim 9, wherein the separated DNA fragments are probed with a labelled Bam H1-generated 1.6 kb fragment of $\lambda hD_2G1$.

11. The method for diagnosing susceptibility to alcoholism as recited in claim 9, wherein the separated fragments are probed with a labelled 30 bit fragment of $\lambda hD_2G1$.

12. A process for diagnosing susceptibility to alcoholism comprising:

detecting in a sample from an individual a dopamin $D_2$ receptor gene characteristically present in clinically diagnosed alcoholic persons by detecting the dopamin $D_2$ receptor gene allele A1 which is identifiable by presence of a 6.6 kb TagI fragment, wherein detection of the A1 allele is accomplished by hybridizing DNA of a sample from the individual to a dopamin $D_2$ receptor gene probe.

13. A labelled probe specifically binding human dopamin $D_2$ receptor gene A1 allele, said allele being identifiable by presence of a 6.6 kb Tag I fragment, for use in a diagnosis of the susceptibility to alcoholism.

14. A labelled probe according to claim 13 specifically binding an allele of human $D_2$ receptor gene obtained after Tag I hydrolysis of human DNA, for use in a diagnosis of susceptibility to alcoholism.

15. A labelled 1.6 kb probe specifically binding a human $D_2$ receptor gene allele obtained after Tag I hydrolysis of human DNA, for use in a diagnosis of susceptibility to alcoholism.

## Patentansprüche

1. Verfahren zum Diagnostizieren einer Empfänglichkeit für Alkoholismus, wobei das Verfahren das Nachweisen eines humanen Dopamin D2-Rezeptorgenallels, das in einer Probe eines Individuums vorhanden ist, durch Hybridisieren der DNA einer Probe von einem Individuum mit einer Dopamin $D_2$-Rezeptorgensonde umfaßt;
wobei die Empfänglichkeit für Alkoholismus eines Individuums diagnostiziert werden kann, wenn das humane Dopamin $D_2$-Rezeptorgen Allel A1 vorhanden ist, das durch die Gegenwart eines 6,6 kb TagI-Fragmentes identifizierbar ist;
wobei das A1-Allel in einer Vielzahl klinisch diagnostizierter Alkoholiker vorhanden ist.

2. Verfahren zum Diagnostizieren einer Empfänglichkeit für Alkoholismus gemäß Anspruch 1, wobei das Verfahren das Bestimmen von in einer Probe von einem Individuum vorhandenen humanen $D_2$-Rezeptorgenallelen umfaßt;
wobei die Empfänglichkeit für Alkoholismus eines Individuums diagnostiziert werden kann, wenn das A1-Allel gefunden wird.

3. Verfahren zum Diagnostizieren der Empfänglichkeit für Alkoholismus gemäß einem der Ansprüche 1 oder 2, wobei das Verfahren umfaßt:

das Abbauen von DNA eines Subjektes mit einer Restriktionsendonuklease;

das Bestimmen von in der abgebauten DNA vorhandenem humanem $D_2$-Rezeptorgelallel; und

wobei eine Empfänglichkeit für Alkoholismus diagnostiziert wird, wenn ein bestimmtes Allel vorhanden ist,

welches in einer Vielzahl von klinisch diagnostizierten Alkoholikern gefunden wird.

4. Verfahren zum Diagnostizieren einer Empfänglichkeit für Alkoholismus gemäß Anspruch 3, wobei das Verfahren umfaßt

das Abbauen von DNA eines Subjektes mit Restriktionsendonuklease $\underline{Taq}$I;

das Bestimmen der Gegenwart von humanen $D_2$-Rezeptorgenallelen in der abgebauten DNA;

wobei eine Empfänglichkeit für Alkoholismus diagnostiziert werden kann, wenn ein besonderes Allel, das in einer Vielzahl von klinisch diagnostizierten Alkoholikern gefunden wird, vorhanden ist.

5. Verfahren zum Diagnostizieren einer Empfänglichkeit für Alkoholismus gemäß Anspruch 3, wobei das Verfahren umfaßt

das Abbauen von DNA eines Subjektes mit Restriktionsendonuklease $\underline{Taq}$I;

das Auftrennen von in dem Abbauansatz gefundenen DNA-Fragmenten;

das Sondieren getrennter Fragmente mit markiertem $\lambda hD_2G1$ oder Fragmenten davon mit ungefähr 30 Bits, um die Gegenwart von humanen $D_2$-Rezeptorgenallelen in der Probe zu bestimmen; und

wobei eine Empfänglichkeit für Alkoholismus diagnostiziert werden kann, wenn ein besonderes Allel vorhanden ist, das dadurch gekennzeichnet ist, daß es in einer Vielzahl von klinisch diagnostizierten Alkoholikern vorhanden ist.

6. Verfahren zum Nachweisen einer Empfänglichkeit für Alkoholismus nach einem der Ansprüche 1, 3 oder 4, wobei das humane $D_2$-Rezeptorgenallel ein humanes $D_2$-Rezeptorgen-A1-Allel ist.

7. Verfahren zum Diagnostizieren einer Empfänglichkeit für Alkoholismus gemäß Anspruch 5, wobei die getrennten Fragmente mit einem von $\underline{Bam}$HI erzeugten 1,6 kb-Fragment von $\lambda hD_2G1$ sondiert werden.

8. Verfahren zum Diagnostizieren einer Empfänglichkeit für Alkoholismus gemäß Anspruch 5, wobei die getrennten DNA-Fragmente mit einem 30 Bit-Fragment von $\lambda hD_2G1$ sondiert werden.

9. Verfahren zum Diagnostizieren einer Empfänglichkeit für Alkoholismus gemäß Anspruch 3, wobei das Verfahren umfaßt:

das Abbauen von DNA eines Subjektes mit Restriktionsendonuklease $\underline{Taq}$I;

das Auftrennen der aus dem Hydrolysat erhaltenen DNA-Fragmente gemäß der Fragmentgröße;

das Sondieren getrennter DNA-Fragmente mit markiertem $\lambda hD_2G1$ oder einem Teil von markiertem $\lambda hD_2G1$ mit einer Größe von mindestens 30 Bits;

das Bestimmen der Gegenwart eines 6,6 kb-DNA-Fragmentes in den getrennten Fragmenten, das durch die Sonde markiert wird; und

wobei eine Empfänglichkeit für Alkoholismus diagnostiziert werden kann, wenn das markierte 6,6 kb-Fragment gefunden wird.

10. Verfahren zum Diagnostizieren einer Empfänglichkeit für Alkoholismus nach Anspruch 9, wobei die getrennten DNA-Fragmente mit einem markierten $\underline{Bam}$HI-erzeugten 1,6 kb-Fragment von $\lambda hD_2G1$ sondiert werden.

11. Verfahren zum Diagnostizieren einer Empfänglichkeit für Alkoholismus nach Anspruch 9, wobei die getrennten Fragmente mit einem markierten 30 Bit-Fragment von $\lambda hD_2G1$ sondiert werden.

12. Verfahren zum Diagnostizieren einer Empfänglichkeit für Alkoholismus, das das Nachweisen eines Dopamin-$D_2$-

Rezeptorgenes in einer Probe von einem Individuum, das charakteristischerweise in klinisch diagnostizierten alkoholabhängigen Personen vorhanden ist, durch Nachweisen des Dopamin-$D_2$-Rezeptorgenalleles A1, das durch die Gegenwart eines 6,6 kb-<u>Taq</u>I-Fragmentes identifizierbar ist, wobei der Nachweis des A1-Allels durch Hybridisieren der DNA von einer Probe aus dem Individuum mit einer Dopamin-$D_2$-Rezeptorgenprobe erbracht wird, umfaßt.

13. Markierte Probe, die spezifisch an das humane Dopamin-$D_2$-Rezeptorgenallel A1 bindet, wobei das Allel durch die Gegenwart eines 6,6 kb-<u>Taq</u>I-Fragmentes indentifizierbar ist, zur Verwendung in der Diagnose der Empfänglichkeit für Alkoholismus.

14. Markierte Probe gemäß Anspruch 13, die spezifisch ein Allel eines humanen $D_2$-Rezeptorgenes bindet, das nach <u>Taq</u>I-Hydrolyse humaner DNA erhalten wird, zur Verwendung in der Diagnose einer Empfänglichkeit für Alkoholismus.

15. Markierte 1,6 kb-Sonde, die spezifisch ein humanes $D_2$-Rezeptorgenallel bindet, das nach <u>Taq</u>I-Hydrolyse von humaner DNA erhalten wird, zur Verwendung bei der Diagnose einer Empfänglichkeit für Alkoholismus.

**Revendications**

1. Procédé pour diagnostiquer la prédisposition à l'alcoolisme, ce procédé comprenant :

la détection de l'allèle du gène récepteur de dopamine humaine $D_2$ présent dans un échantillon provenant d'un individu, en hybridant l'ADN d'un échantillon provenant d'un sujet à une sonde de gène récepteur de dopamine $D_2$ ;
dans lequel la prédisposition à l'alcoolisme d'un individu peut être diagnostiquée, quand l'allèle A1 du gène récepteur de dopamine humaine $D_2$, identifiable par la présence d'un fragment <u>Taq</u> I de 6,6 kb, est présent ;
dans lequel l'allèle A1 est présent chez une majorité d'alcooliques cliniquement diagnostiqués.

2. Procédé pour diagnostiquer la prédisposition à l'alcoolisme selon la revendication 1, ce procédé comprenant :

la détermination de la présence d'allèles du gène récepteur de $D_2$ humaine dans un échantillon provenant d'un individu ;
où la prédisposition d'un individu à l'alcoolisme peut être diagnostiquée quand on trouve l'allèle A1.

3. Procédé pour diagnostiquer la prédisposition à l'alcoolisme selon l'une quelconque des revendications 1 ou 2, ce procédé comprenant :

la digestion de l'ADN provenant d'un sujet avec une endonucléase de restriction ;
la détermination de la présence d'un allèle du gène récepteur de $D_2$ humaine dans ledit ADN digéré ; et
une prédisposition à l'alcoolisme étant diagnostiquée quand un allèle particulier est présent,
que l'on trouve chez une majorité d'alcooliques cliniquement diagnostiqués.

4. Procédé pour diagnostiquer la prédisposition à l'alcoolisme selon la revendication 3, ce procédé comprenant :

la digestion de l'ADN provenant d'un sujet avec l'endonucléase de restriction <u>Taq</u> I ;
la détermination de la présence d'allèles du gène récepteur de $D_2$ humaine dans ledit ADN digéré ;
dans lequel une prédisposition à l'alcoolisme peut être diagnostiquée quand un allèle particulier, que l'on trouve chez une majorité d'alcooliques cliniquement diagnostiqués, est présent.

5. Procédé pour diagnostiquer la prédisposition à l'alcoolisme selon la revendication 3, ce procédé comprenant :

la digestion de l'ADN provenant d'un sujet avec l'endonucléase de restriction <u>Taq</u> I ;
la séparation des fragments d'ADN présents dans le digestat ;
le sondage des fragments séparés avec un $\lambda hD_2G1$ marqué ou des fragments de celui-ci ayant environ 30 bits pour déterminer la présence d'allèles du gène récepteur de $D_2$ humaine dans ledit échantillon ; et
dans lequel la prédisposition à l'alcoolisme peut être diagnostiquée quand un allèle particulier caractérisé par le fait qu'on le trouve chez une majorité d'alcooliques cliniquement diagnostiqués, est présent.

**6.** Procédé pour diagnostiquer la prédisposition à l'alcoolisme selon l'une quelconque des revendications 1, 3 ou 4, dans lequel l'allèle du gène récepteur de $D_2$ humaine est un allèle A1 du gène récepteur de $D_2$ humaine.

**7.** Procédé pour diagnostiquer la prédisposition à l'alcoolisme selon la revendication 5, dans lequel lesdits fragments séparés sont sondés avec un fragment de $\lambda hD_2G1$ de 1,6 kb généré par <u>Bam</u> H1.

**8.** Procédé pour diagnostiquer la prédisposition à l'alcoolisme selon la revendication 5, dans lequel lesdits fragments d'ADN séparés sont sondés avec un fragment de $\lambda hD_2G1$ de 30 bits.

**9.** Procédé pour diagnostiquer la prédisposition à l'alcoolisme selon la revendication 3, ce procédé comprenant :

la digestion de l'ADN provenant d'un sujet avec l'endonucléase de restriction <u>Tag</u> I ;
la séparation des fragments d'ADN obtenus à partir de l'hydrolysat en fonction de la taille des fragments ;
le sondage des fragments d'ADN séparés avec un $\lambda hD_2G1$ marqué ou une partie d'un $\lambda hD_2G1$ marqué ayant une taille d'au moins 30 bits ;
la détermination de la présence d'un fragment d'ADN de 6,6 kb dans lesdits fragments séparés qui est marqué par la sonde ; et
où une prédisposition à l'alcoolisme peut être diagnostiquée quand on trouve ledit fragment de 6,6 kb.

**10.** Procédé pour diagnostiquer de la prédisposition à l'alcoolisme selon la revendication 9, dans lequel les fragments d'ADN séparés sont sondés avec un fragment de $\lambda hD_2G1$ marqué de 1,6 kb généré par <u>Bam</u> H1.

**11.** Procédé pour diagnostiquer la prédisposition à l'alcoolisme selon la revendication 9, dans lequel les fragments séparés sont sondés avec un fragment de $\lambda\ hD_2G1$ marqué de 30 bits.

**12.** Procédé pour diagnostiquer la prédisposition à l'alcoolisme comprenant :

la détection dans un échantillon provenant d'un individu d'un gène récepteur de dopamine $D_2$ présent de manière caractéristique chez les personnes alcooliques cliniquement diagnostiqués en détectant l'allèle A1 du gène récepteur de dopamine $D_2$ qui est identifiable par la présence d'un fragment <u>Tag</u> I de 6,6 kb, dans lequel la détection de l'allèle A1 est réalisée en hybridant l'ADN d'un échantillon provenant de l'individu à une sonde du gène récepteur de dopamine $D_2$.

**13.** Sonde marquée se fixant spécifiquement sur l'allèle A1 du gène récepteur de dopamine $D_2$ humaine, ledit allèle étant identifiable par la présence d'un fragment <u>Tag</u> I de 6,6 kb, pour utilisation dans un diagnostic de prédisposition à l'alcoolisme.

**14.** Sonde marquée selon la revendication 13, se fixant spécifiquement sur un allèle du gène récepteur de $D_2$ humaine obtenu après hydrolyse par <u>Tag</u> I d'ADN humain, pour utilisation dans un diagnostic de prédisposition à l'alcoolisme.

**15.** Sonde marquée de 1,6 kb, se fixant spécifiquement sur un allèle du gène récepteur de $D_2$ humaine obtenu après hydrolyse par <u>Tag</u> I d'ADN humain, pour utilisation dans un diagnostic de prédisposition à l'alcoolisme.

# FIG.1A

# FIG.1B

A2/A2          A1/A2

# FIG.6

A₂  A₁     A₂  A₁

10.5 Kb —

— 6.6 Kb

— 3.7 Kb

CLONE 16     CLONE 9

FIG.1C

FIG.2

FIG.3

# FIG.4A

# FIG.4AA

# FIG.4B

# FIG.4BB

FIG.5

DOUBLET
1.73Kb

FIG.7A

CGCAAGGCCTTCCTGAAGATCCTCCACTGC
||||||||||||||  |||||||||| | ||||||||
CGCAAGGCCTTCATGAAGATCTTGCACTGC

FIG.7B

CLONE 9     CLONE 16

EP 0 514 490 B1